# EUROPEAN PATENT SPECIFICATION

(11) **EP 2 611 428 B1**
(45) Date of publication and mention of the grant of the patent: **14.01.2015**
(21) Application number: 11761000.6
(22) Date of filing: 01.09.2011
(51) Int. Cl.: A61K 9/20, A61K 9/48

(54) **TAMPER RESISTANT DOSAGE FORM COMPRISING AN ANIONIC POLYMER**
MANIPULATIONSSICHERE DARREICHUNGSFORM MIT EINEM ANIONISCHEN POLYMER
FORME PHARMACEUTIQUE INVIOLABLE COMPORTANT UN POLYMERE ANIONIQUE

(30) Priority: 02.09.2010 EP 10009124
(43) Date of publication of application: 10.07.2013
(73) Proprietor: Grünenthal GmbH, 52078 Aachen (DE)
(72) Inventor: BARNSCHEID, Lutz, 41239 Mönchengladbach (DE); SCHWIER, Sebastian, 52076 Aachen (DE); BARTHOLOMÄUS, Johannes, 52080 Aachen (DE)
(74) Representative: Bülle, Jan
(86) International application number: PCT/EP2011/004404
(87) International publication number: WO 2012/028317

(56) References cited:
- EP-A2- 1 842 533
- WO-A2-2007/085024
- WO-A2-2008/107149
- US-A1- 2005 031 546

## Description

The invention relates to a pharmaceutical dosage form exhibiting a breaking strength of at least 500 N, said dosage form containing a pharmacologically active ingredient (A); an anionic polysaccharide (B) obtainable by introducing anionic functional groups, in protonated form or a physiologically acceptable salt thereof, into a polysaccharide; and a polyalkylene oxide (C); wherein the ingredient (A) is present in a controlled-release matrix comprising the anionic polysaccharide (B) and the polyalkylene oxide (C).

Many pharmacologically active ingredients have a potential of being abused and thus, are advantageously provided in form of tamper resistant pharmaceutical dosage forms. Prominent examples of such pharmacologically active ingredients are opioids.

It is known that abusers crush conventional tablets, which contain opioids, to defeat the time-release "micro-encapsulation" and then ingest the resulting powder orally, intra-nasally, rectally, or by injection.

Various concepts for the avoidance of drug abuse have been developed. One concept relies on the mechanical properties of the pharmaceutical dosage forms, particularly an increased breaking strength (resistance to crushing). The major advantage of such pharmaceutical dosage forms is that comminuting, particularly pulverization, by conventional means, such as grinding in a mortar or fracturing by means of a hammer, is impossible or at least substantially impeded.

Such pharmaceutical dosage forms are useful for avoiding drug abuse of the pharmacologically active ingredient contained therein, as they may not be powdered by conventional means and thus, cannot be administered in powdered from, e.g. nasally. The mechanical properties, particularly the high breaking strength of these pharmaceutical dosage forms renders them tamper resistant. In the context of such tamper resistant pharmaceutical dosage forms it can be referred to, e.g., US 2005/031546, WO 2005/016313, WO 2005/016314, WO 2005/ 063214, WO 2005/102286, WO 2006/002883, WO 2006/002884, WO 2006/002886, WO 2006/082097, WO 2006/082099, WO 2008/107149, and WO 2009/092601.

WO 2007/085024 discloses a dosage form and method for the delivery of drugs, particularly drugs of abuse, characterized by resistance to solvent extraction, tampering, crushing, or grinding, and providing an initial burst of release of drug followed by a prolonged period of controllable drug release.

The release kinetics of the pharmacologically active ingredients from such tamper resistant dosage forms is an important factor. It is well known that depending on how a pharmaceutically active ingredient is formulated into a tablet its release pattern can be modified. In this regard, tablets providing a retarded release profile are of primary importance. With retarded release tablets care has to be taken that under no circumstances the pharmaceutically active ingredient will be released completely and instantaneously in an uncontrolled manner ("dose-dumping") since regularly the dosage used for retarded release tablets is much higher than for non-retarded release tablets. This may cause serious adverse effects or even death depending on the active ingredient and potency thereof.

Controlled release (e.g. delayed release, prolonged release, sustained release, and the like) may be based upon various concepts such as coating the pharmaceutical dosage form with a controlled release membrane, embedding the pharmacologically active ingredient in a matrix, binding the pharmacologically active ingredient to an ion-exchange resin, forming a complex of the pharmacologically active ingredient, and the like. In this context it can be referred to, e.g., W.A. Ritschel, Die Tablette, 2. Auflage, Editio Cantor Verlag Aulendorf, 2002.

It is well known that a pharmaceutical formulation or its mode of manufacture, e.g. for an oral dosage form, might undergo modifications during clinical testing, for example with respect to the ingredients used or to the relative amounts of the excipients, or with respect to the reaction conditions and reactants used during manufacture. Frequently, such modifications at least to some extent have an impact on the release profile of pharmaceutically active ingredients. This is particularly unpleasant if for a specific formulation an approved optimized release profile has already been found which cannot be reproduced with the modified formulation. In such a case, the clinical tests have either to be interrupted or have to be started from the beginning. Given the huge expenditures necessary to bring a new drug formulation up to and through clinical testing the above scenario has indeed proven to be rather unsatisfactory.

Particular problems arise when the dose of the pharmacologically active ingredient and thus, also the total weight of the pharmaceutical dosage form is comparatively high. Depending upon the content and the nature of the pharmacologically active ingredient and of the pharmaceutical excipients, the retardant effect of the polymer may be too weak so that the pharmaceutical dosage form cannot be adapted to a specific dosing regimen, e.g., twice daily, particularly when the increased breaking strength is to be maintained.

An increase of the content of the retardant polymer for the purpose of decelerating drug release would substantially increase the total weight of the pharmaceutical dosage form and in a worst case scenario, would lead to a size that could not be swallowed by a subject.

Thus, there is a demand for tamper-resistant pharmaceutical dosage forms, the release profile of which may be varied within certain limits without diminishing the tamper resistance and without deteriorating the compliance of the pharmaceutical dosage form.

It is an object of the invention to provide pharmaceutical dosage forms having advantages compared to pharmaceutical dosage forms of the prior art.

This object has been achieved by the subject-matter of the patent claims.

It has been surprisingly found that comparatively low amounts of matrix polymers bearing anionic functional groups provide a further delay of the release of the pharmacologically active ingredients from the tamper resistant dosage forms without diminishing the specific mechanical properties and without leading to a substantial increase of the overall weight.

Further, it has been surprisingly found that said matrix polymers bearing anionic functional groups can even improve the mechanical properties of the dosage forms.
Figure 1 shows the in vitro release profile of a pharmaceutical dosage form according to the invention containing 10 wt-% respectively 20 wt-% of sodium carboxymethylstarch.
Figure 2 shows the in vitro release profiles of a pharmaceutical dosage form according to the invention containing 10 wt-%; 20 wt-% of carmellose sodium respectively 10 wt-% of carmellose sodium and 10 wt-% of hypromellose.
Figure 3 shows the in vitro release profiles of a pharmaceutical dosage form according to the invention containing 10 wt-%; 20 wt-% of carmellose respectively 10 wt-% of carmellose and 10 wt-% of hypromellose.
Figure 4 shows the in vitro release profiles of a pharmaceutical dosage form according to the invention containing 10 wt-%; 20 wt-% of carmellose respectively 10 wt-% of carmellose and 10 wt-% of hypromellose in acid media with a pH of 1.2.
Figure 5 shows the in vitro release profiles of a pharmaceutical dosage form according to the invention containing 10 wt-%; 20 wt-% of crosscarmellose sodium respectively 10 wt-% of crosscarmellose sodium and 10 wt-% of hypromellose.
Figure 6 shows the in vitro release profiles of a pharmaceutical dosage form according to the invention containing 10 wt-% of κ-carrageenan againts 10 wt-% hypromellose..
Figure 7 shows the in vitro release profile of a comparative pharmaceutical dosage form containing 10 wt-% respectively 20 wt-% of corn starch.
Figure 8 shows the in vitro release profile of a comparative pharmaceutical dosage form containing 10 wt-% respectively 20 wt-% of pregelatinized starch.
Figure 9 shows the in vitro release profile of a comparative pharmaceutical dosage form containing 10 wt-% of cellulose acetate.
Figure 10 shows the in vitro release profile of a comparative pharmaceutical dosage form containing 10 wt-% of pectin.
Figure 11 shows the in vitro release profile of a comparative pharmaceutical dosage form containing 10 wt-% respectively 20 wt-% of phosphatized starch respectively 10 wt-% phosphatized starch and 10 wt-% hypromellose.
A first aspect of the invention relates to a pharmaceutical dosage form exhibiting a breaking strength of at least 500 N, said dosage form containing
   - a pharmacologically active ingredient (A);
   - an anionic polysaccharide (B) obtainable by introducing anionic functional groups, in protonated form or a physiologically acceptable salt thereof, into a polysaccharide; and
   - a polyalkylene oxide (C);
wherein the pharmacologically active ingredient (A) is present in a controlled-release matrix comprising the anionic polysaccharide (B) and the polyalkylene oxide (C).

The dosage form according to the invention contains one or more pharmacologically active ingredients (A).

There are generally no limitations as to the pharmacologically active ingredient (A) (pharmacologically active compound) which can be incorporated into the tablet of the invention.

In a preferred embodiment, the pharmaceutical dosage form contains only a single pharmacologically active ingredient (A). In another preferred embodiment, the pharmaceutical dosage form contains a combination of two or more pharmacologically active ingredients (A).

Preferably, pharmacologically active ingredient (A) has potential for being abused. Active ingredients with potential for being abused are known to the person skilled in the art and comprise e.g. tranquillisers, stimulants, barbiturates, narcotics, opioids or opioid derivatives.

Preferably, the pharmacologically active ingredient (A) exhibits psychotropic action.

Preferably, the pharmacologically active ingredient (A) is selected from the group consisting of opiates, opioids, stimulants, tranquilizers, and other narcotics.

Particularly preferably, the pharmacologically active ingredient (A) is an opioid. According to the ATC index, opioids are divided into natural opium alkaloids, phenylpiperidine derivatives, diphenylpropylamine derivatives, benzomorphan derivatives, oripavine derivatives, morphinan derivatives and others.

The following opiates, opioids, tranquillizers or other narcotics are substances with a psychotropic action, i.e. have a potential of abuse, and hence are preferably contained in the pharmaceutical dosage form according to the invention: alfentanil, allobarbital, allylprodine, alphaprodine, alprazolam, amfepramone, amphetamine, amphetaminil, amobarbital, anileridine, apocodeine, axomadol, barbital, bemidone, benzylmorphine, bezitramide, bromazepam, brotizolam, buprenorphine, butobarbital, butorphanol, camazepam, carfentanil, cathine/D-norpseudoephedrine, chlordiazepoxide, clobazam clofedanol, clonazepam, clonitazene, clorazepate, clotiazepam, cloxazolam, cocaine, codeine, cyclobarbital, cyclorphan, cyprenorphine, delorazepam, desomorphine, dextromoramide, dextropropoxyphene, dezocine, diampromide, diamorphone, diazepam, dihydrocodeine, dihydromorphine, dihydromorphone, dimenoxadol, dimephetamol, dimethylthiambutene, dioxaphetylbutyrate, dipipanone, dronabinol, eptazocine, estazolam, ethoheptazine, ethylmethylthiambutene, ethyl loflazepate, ethylmorphine, etonitazene, etorphine, faxeladol, fencamfamine, fenethylline, fenpipramide, fenproporex, fentanyl, fludiazepam, flunitrazepam, flurazepam, halazepam, haloxazolam, heroin, hydrocodone, hydromorphone, hydroxypethidine, isomethadone, hydroxymethylmorphinan, ketazolam, ketobemidone, levacetylmethadol (LAAM), levomethadone, levorphanol, levophenacylmorphane, levoxemacin, lisdexamfetamine dimesylate, lofentanil, loprazolam, lorazepam, lormetazepam, mazindol, medazepam, mefenorex, meperidine, meprobamate, metapon, meptazinol, metazocine, methylmorphine, metamphetamine, methadone, methaqualone, 3-methylfentanyl, 4-methylfentanyl, methylphenidate, methylphenobarbital, methyprylon, metopon, midazolam, modafinil, morphine, myrophine, nabilone, nalbuphene, nalorphine, narceine, nicomorphine, nimetazepam, nitrazepam, nordazepam, norievorphanol, normethadone, normorphine, norpipanone, opium, oxazepam, oxazolam, oxycodone, oxymorphone, Papaver somniferum, papaveretum, pernoline, pentazocine, pentobarbital, pethidine, phenadoxone, phenomorphane, phenazocine, phenoperidine, piminodine, pholcodeine, phenmetrazine, phenobarbital, phentermine, pinazepam, pipradrol, piritramide, prazepam, profadol, proheptazine, promedol, properidine, propoxyphene, remifentanil, secbutabarbital, secobarbital, sufentanil, tapentadol, temazepam, tetrazepam, tilidine (cis and trans), tramadol, triazolam, vinylbital, N-(1-methyl-2-piperidinoethyl)-N-(2-pyridyl)propionamide, (1R,2R)-3-(3-dimethylamino-1-ethyl-2-methyl-propyl)phenol, (1R,2R,4S)-2-(dimethylamino)-methyl-4-(p-fluorobenzyloxy)-1-(m-methoxyphenyl)cyclohexanol, (1R,2R)-3-(2-dimethylaminomethyl-cyclohexyl)phenol, (1S,2S)-3-(3-dimethylamino-1-ethyl-2-methyl-propyl)phenol, (2R,3R)-1-dimethylamino-3(3-methoxyphenyl)-2-methyl-pentan-3-ol, (1RS,3RS,6RS)-6-dimethylaminomethyl-1-(3-methoxyphenyl)-cyclohexane-1,3-diol, preferably as racemate, 3-(2-dimethylaminomethyl-1-hydroxy-cyclohexyl)phenyl 2-(4-isobutyl-phenyl)propionate, 3-(2-dimethylaminomethyl-1-hydroxy-cyclohexyl)phenyl 2-(6-methoxy-naphthalen-2-yl)propionate, 3-(2-dimethylaminomethyl-cyclohex-1-enyl)-phenyl 2-(4-isobutyl-phenyl)propionate, 3-(2-dimethylaminomethyl-cyclohex-1-enyl)-phenyl 2-(6-methoxy-naphthalen-2-yl)propionate, (RR-SS)-2-acetoxy-4-trifluoromethyl-benzoic acid 3-(2-dimethylaminomethyl-1-hydroxycyclohexyl)-phenyl ester, (RR-SS)-2-hydroxy-4-trifluoromethyl-benzoic acid 3-(2-dimethylaminomethyl-1-hydroxy-cyclohexyl)-phenyl ester, (RR-SS)-4-chloro-2-hydroxybenzoic acid 3-(2-dimethylaminomethyl-1-hydroxy-cyclohexyl)-phenyl ester, (RR-SS)-2-hydroxy-4-methyl-benzoic acid 3-(2-dimethylaminomethyl-1-hydroxy-cyclohexyl)-phenyl ester, (RR-SS)-2-hydroxy-4-methoxy-benzoic acid 3-(2-dimethylaminomethyl-1-hydroxy-cyclohexyl)-phenyl ester, (RR-SS)-2-hydroxy-5-nitro-benzoic acid 3-(2-dimethylaminomethyl-1-hydroxy-cyclohexyl)-phenyl ester, (RR-SS)-2',4'-difluoro-3-hydroxy-biphenyl-4-carboxylic acid 3-(2-dimethylaminomethyl-1-hydroxy-cyclohexyl)-phenyl ester, and corresponding stereoisomeric compounds, in each case the corresponding derivatives thereof, physiologically acceptable enantiomers, stereoisomers, diastereomers and racemates and the physiologically acceptable derivatives thereof, e.g. ethers, esters or amides, and in each case the physiologically acceptable compounds thereof, in particular the acid or base addition salts thereof and solvates, e.g. hydrochlorides.

In a preferred embodiment the pharmaceutical dosage form according to the invention contains an opioid selected from the group consisting of DPI-125, M6G (CE-04-410), ADL-5859, CR-665, NRP290 and sebacoyl dinalbuphine ester.

In a preferred embodiment, the pharmaceutical dosage form according to the invention contains one pharmacologically active ingredient (A) or more pharmacologically active ingredients (A) selected from the group consisting of oxymorphone, hydromorphone and morphine.

In another preferred embodiment, the pharmacologically active ingredient (A) is selected from the group consisting of tapentadol, faxeladol and axomadol.

In still another preferred embodiment, the pharmacologically active ingredient (A) is selected from the group consisting of 1,1-(3-dimethylamino-3-phenylpentamethylene)-6-fluoro-1,3,4,9-tetrahydropyrano[3,4-b]indole, particularly its hemicitrate; 1,1-[3-dimethylamino-3-(2-thienyl)-pentamethylene]-1,3,4,9-tetrahydropyrano[3,4-b]indole, particularly its citrate; and 1,1-[3-dimethylamino-3-(2-thienyl)pentamethylene]-1,3,4,9-tetrahydropyrano[3,4-b]-6-fluoroindole, particularly its hemicitrate. These compounds are known from, e.g., WO 2004/043967, WO 2005/066183.

The pharmacologically active ingredient (A) may be present in form of a physiologically acceptable salt, e.g. physiologically acceptable acid addition salt.

Physiologically acceptable acid addition salts comprise the acid addition salt forms which can conveniently be obtained by treating the base form of the active ingredient with appropriate organic and inorganic acids. Active ingredients containing an acidic proton may be converted into their non-toxic metal or amine addition salt forms by treatment with appropriate organic and inorganic bases. The term addition salt also comprises the hydrates and solvent addition forms which the active ingredients are able to form. Examples of such forms are e.g. hydrates, alcoholates and the like.

The pharmacologically active ingredient (A) is present in the dosage form in a therapeutically effective amount. The amount that constitutes a therapeutically effective amount varies according to the active ingredients being used, the condition being treated, the severity of said condition, the patient being treated, and whether the dosage form is designed for an immediate or retarded release. The amount of active ingredient(s) used in the present invention preferably ranges from about 0.01 wt-% to about 95 wt%, more preferably from about 0.1 wt-% to about 80 wt-%, even more preferably from about 1.0 wt-% to about 50 wt-%, yet more preferably from about 1.5 wt-% to about 30 wt-%, and most preferably from about 2.0 wt-% to 20 wt-%.

The content of the pharmacologically active ingredient (A) in the pharmaceutical dosage form is not limited. The dose of the pharmacologically active ingredient (A) which is adapted for administration preferably is in the range of 0.1 mg to 500 mg, more preferably in the range of 1.0 mg to 400 mg, even more preferably in the range of 5.0 mg to 300 mg, and most preferably in the range of 10 mg to 250 mg. In a preferred embodiment, the total amount of the pharmacologically active ingredient (A) that is contained in the pharmaceutical dosage form is within the range of from 0.01 to 200 mg, more preferably 0.1 to 190 mg, still more preferably 1.0 to 180 mg, yet more preferably 1.5 to 160 mg, most preferably 2.0 to 100 mg and in particular 2.5 to 80 mg.

Preferably, the content of the pharmacologically active ingredient (A) is within the range of from 0.01 to 80 wt.-%, more preferably 0.1 to 50 wt.-%, still more preferably 1 to 25 wt.-%, based on the total weight of the pharmaceutical dosage form. In a preferred embodiment, the content of pharmacologically active ingredient (A) is within the range of from 7±6 wt.-%, more preferably 7±5 wt.-%, still more preferably 5±4 wt.-%, 7±4 wt.-% or 9±4 wt.-%, most preferably 5±3 wt.-%, 7±3 wt.-% or 9±3 wt.-%, and in particular 5±2 wt.-%, 7±2 wt.-% or 9±2 wt.-%, based on the total weight of the pharmaceutical dosage form. In another preferred embodiment, the content of pharmacologically active ingredient (A) is within the range of from 11±10 wt.-%, more preferably 11±9 wt.-%, still more preferably 9±6 wt.-%, 11±6 wt.-%, 13±6 wt.-% or 15±6 wt.-%, most preferably 11±4 wt.-%, 13±4 wt.-% or 15±4 wt.-%, and in particular 11±2 wt.-%, 13±2 wt.-% or 15±2 wt.-%, based on the total weight of the pharmaceutical dosage form. In a further preferred embodiment, the content of pharmacologically active ingredient (A) is within the range of from 20±6 wt.-%, more preferably 20±5 wt.-%, still more preferably 20±4 wt.-%, most preferably 20±3 wt.-%, and in particular 20±2 wt.-%, based on the total weight of the pharmaceutical dosage form.

In a preferred embodiment, the pharmacologically active ingredient (A) is contained in the pharmaceutical dosage form in an amount of 7.5±5 mg, 10±5 mg, 20±5 mg, 30±5 mg, 40±5 mg, 50±5 mg, 60±5 mg, 70±5 mg, 80±5 mg, 90±5 mg, 100±5 mg, 110±5 mg, 120±5 mg. 130±5. 140±5 mg, 150±5 mg, or 160±5 mg. In another preferred embodiment, the pharmacologically active ingredient (A) is contained in the pharmaceutical dosage form in an amount of 5±2.5 mg, 7.5±2.5 mg, 10±2.5 mg, 15±2.5 mg, 20±2.5 mg, 25±2.5 mg, 30±2.5 mg, 35±2.5 mg, 40±2.5 mg, 45±2.5 mg, 50±2.5 mg, 55±2.5 mg, 60±2.5 mg, 65±2.5 mg, 70±2.5 mg, 75±2.5 mg, 80±2.5 mg, 85±2.5 mg, 90±2.5 mg, 95±2.5 mg, 100±2.5 mg, 105±2.5 mg, 110±2.5 mg, 115±2.5 mg, 120±2.5 mg, 125±2.5 mg, 130±2.5 mg, 135±2.5 mg, 140±2.5 mg, 145±2.5 mg, 150±2.5 mg, 155±2.5 mg, or 160±2.5 mg.

Preferably the pharmaceutically dosage form provides a release of the pharmacologically active ingredient (A) after 1 hour of preferably at most 60 %, more preferably at most 40 %, yet more preferably at most 30 %, still more preferably at most 20 % and most preferably at most 17%. After 2 hour preferably at most 80 %, more preferably at most 60 %, yet more preferably at most 50 %, still more preferably at most 40 % and most preferably at most 32%. After 3 hour preferably at most 85 %, more preferably at most 65 %, yet more preferably at most 55 %, still more preferably at most 48 % and most preferably at most 42%. After 4 hour preferably at most 90 %, more preferably at most 75 %, yet more preferably at most 65 %, still more preferably at most 55 % and most preferably at most 49%. After 7 hour preferably at most 95 %, more preferably at most 85 %, yet more preferably at most 80 %, still more preferably at most 70 % and most preferably at most 68%. After 10 hour preferably at most 99 %, more preferably at most 90 %, yet more preferably at most 88 %, still more preferably at most 83 % and most preferably at most 80%. After 13 hour preferably at most 99 %, more preferably at most 95 %, yet more preferably at most 93 %, still more preferably at most 91 % and most preferably at most 89%.

In a particularly preferred embodiment, pharmacologically active ingredient (A) is tapentadol, preferably its HCl salt, and the pharmaceutical dosage form is adapted for administration once daily or twice daily. In this embodiment, pharmacologically active ingredient (A) is preferably contained in the pharmaceutical dosage form in an amount of from 25 to 250 mg.

In a particularly preferred embodiment, pharmacologically active ingredient (A) is oxymorphone, preferably its HCl salt, and the pharmaceutical dosage form is adapted for administration twice daily. In this embodiment, pharmacologically active ingredient (A) is preferably contained in the pharmaceutical dosage form in an amount of from 5 to 40 mg. In another particularly preferred embodiment, pharmacologically active ingredient (A) is oxymorphone, preferably its HCl, and the pharmaceutical dosage form is adapted for administration once daily. In this embodiment, pharmacologically active ingredient (A) is preferably contained in the pharmaceutical dosage form in an amount of from 10 to 80 mg.

In another particularly preferred embodiment, pharmacologically active ingredient (A) is oxycodone, preferably its HCl salt, and the pharmaceutical dosage form is adapted for administration twice daily. In this embodiment, pharmacologically active ingredient (A) is preferably contained in the pharmaceutical dosage form in an amount of from 5 to 80 mg. In another particularly preferred embodiment, pharmacologically active ingredient (A) is oxycodone, preferably its HCl, and the pharmaceutical dosage form is adapted for administration once daily. In this embodiment, pharmacologically active ingredient (A) is preferably contained in the pharmaceutical dosage form in an amount of from 10 to 320 mg.

In still another particularly preferred embodiment, pharmacologically active ingredient (A) is hydromorphone, preferably its HCl, and the pharmaceutical dosage form is adapted for administration twice daily. In this embodiment, pharmacologically active ingredient (A) is preferably contained in the pharmaceutical dosage form in an amount of from 2 to 52 mg. In another particularly preferred embodiment, pharmacologically active ingredient (A) is hydromorphone, preferably its HCl, and the pharmaceutical dosage form is adapted for administration once daily. In this embodiment, pharmacologically active ingredient (A) is preferably contained in the pharmaceutical dosage form in an amount of from 4 to 104 mg.

The pharmaceutical dosage form according to the invention is characterized by excellent storage stability. Preferably, after storage for 4 weeks at 40°C and 75% rel. humidity, the content of pharmacologically active ingredient (A) amounts to at least 98.0%, more preferably at least 98.5%, still more preferably at least 99.0%, yet more preferably at least 99.2%, most preferably at least 99.4% and in particular at least 99.6%, of its original content before storage. Suitable methods for measuring the content of the pharmacologically active ingredient (A) in the pharmaceutical dosage form are known to the skilled artisan. In this regard it is referred to the Eur. Ph. or the USP, especially to reversed phase HPLC analysis. Preferably, the pharmaceutical dosage form is stored in closed, preferably sealed containers, preferably as described in the experimental section, most preferably being equipped with an oxygen scavenger, in particular with an oxygen scavenger that is effective even at low relative humidity.

The dosage form according to the invention contains an anionic polysaccharide (B) obtainable by introducing anionic functional groups, in protonated form or a physiologically acceptable salt thereof, into a polysaccharide. The active ingredient (A) is embedded into a controlled-release matrix comprising said anionic polysaccharide (B).

For the purpose of the specification, the term "saccharide" and "polysaccharide" respectively, refers not only to the plain (poly-)hydroxycarbonyl compounds but particularly also to their chemically modified derivatives such as esters and ethers. Preferred anionic polysaccharides bear ether groups in the side chain of the polysaccharides which in turn contain anionic functional groups such as -CH₂-CO₂H or ester groups of inorganic multicarboxylic acids such as -OSO₃H.

Preferably, the anionic functional group is selected from carboxyl groups, sulfonyl groups, sulfate groups, and phosphoryl groups, in each case optionally linked to one of the hydroxyl groups of the polysaccharide through -C₁-C₄-alkylene- groups. Carboxyl groups linked through -CH₂- (i.e., -CH₂-CO₂H) and sulfate groups directly esterified (i.e., -OSO₃H) with a hydroxyl group of the polysaccharide are particularly preferred.

In a preferred embodiment, the polysaccharide is a polyhydroxycarbonyl compound not containing functional groups other than primary and secondary hydroxyl groups, carbonyl groups, and acetal and ketal groups, respectively. According to this embodiment, the polysaccharide as such does not contain any anionic acidic functional groups, which in turn are introducable by chemical derivatization of the polysaccharide e.g. by etherification of the primary or secondary hydroxyl groups (e.g., -CH₂CO₂H) or esterification thereof (e.g., -OSO₃H).

Preferably, the anionic polysaccharide (B) is selected from the group consisting of carboxymethylcellulose, carboxymethylcellulose sodium, carboxymethylstarch, sodium carboxymethylstarch, crosscarmellose and carrageenan, preferably κ-carrageenan.

The anionic polysaccharide (B) is principally obtainable by anionically derivatizing an existent polymer (polymer analogous reaction) and/or by polymerization of a monomer composition containing monomers that are already anionically derivatized. This does not necessarily require that the anionic polysaccharide (B) has been obtained by any of these methods. In other words, the anionic polysaccharide (B) is a polymer obtainable by introducing anionic functional groups, in protonated form or a physiologically acceptable salt thereof, into a polysaccharide, or which results from polymerization of a saccharide monomer that is modified after its polymerization by a polymer analogous reaction introducing an anionic functional group to at least one repeating unit.

The anionic polysaccharide (B) may be linear or branched and/or cross-linked.

Preferably, anionic polysaccharide (B) is hydrophilic, more preferably water-soluble or water-swellable.

The anionic polysaccharide (B) may be a homopolymer or a copolymer.

When anionic polysaccharide (B) is a homopolymer, it comprises a single type of repeating unit, e.g. is the polymerization product of a monomer composition comprising a single type of monomer.

When anionic polysaccharide (B) is a copolymer, it may comprise two, three or more different repeating units, i.e. may be the polymerization product of a monomer composition comprising two, three or more different monomers, or comprising a single monomer that can react at various positions so that different repeating units are formed (e.g. glucose in amylopectin).

As anionic polysaccharide (B) is obtainable by introducing anionic functional groups into a polysaccharide, said polysaccharide (starting material) may be e.g. a homopolymer. However, introducing anionic functional groups in the course of a polymer analogous reaction may generate different types of repeating units, particularly repeating units bearing anionic functional groups and repeating units not bearing anionic functional groups. Therefore, by introducing anionic functional groups, a homopolymer (staring material) may become a copolymer (anionic polysaccharide) in the meaning of the invention.

In a preferred embodiment, the anionic polysaccharide (B) is a copolymer, comprising from about 50 mol-% to 99.999 mol%, and more preferably from about 75 mol-% to 99.99 mol-% repeating units bearing anionic functional groups, preferably acidic groups, more preferably carboxylic groups.

In a preferred embodiment, anionic polysaccharide (B) is cross-linked. Cross-linking may be achieved by a cross-linking agent.

Suitable cross-linking agents include compounds having at least two functional groups that are capable of reacting with other functional groups of one or more of the repeating units of anionic polysaccharide (B).

Suitable cross-linking agents having at least two functional groups capable of reacting with other functional groups of one or more of the repeating units of anionic polysaccharide (B) include glyoxal; polyols such as ethylene glycol; polyamines such as alkylene diamines (e.g., ethylene diamine), polyalkylene polyamines, polyepoxides, di- or polyglycidyl ethers and the like.

Suitable cross-linking reactions are the polymerization involving a cross-linker mentioned above and/or the formation of a glycosidic bond. The polymerization preferably takes place at the positions 2, 3, 5 and 6 of a aldohexose or a ketohexose. A glycosidic bond is formed between the hemiacetal group at the anomeric carbon, of a pyranose preferably of glucose, or a molecule derived from a pyranose, and the hydroxyl group of an organic compound such as an alcohol or another pyranose unit.

In a preferred embodiment, the anionic functional groups of the anionic polysaccharide (B) as such do not contribute to cross-linking. Thus, for example, cross-linking is preferably not achieved through phosphate moieties, e.g. by phosphoric acid diesters. Thus, when the anionic functional groups are phosphate groups, these phosphate groups are preferably present as phosphoric acid monoesters, but not as phosphoric acid diesters cross-linking two polymeric backbones with one another.

Cross-linking generates repeating units having at least three linkages to other repeating units. Two of said linkages are needed for the polymer backbone whereas the third linkage constitutes a branching point. For the purpose of the specification, such repeating units are also referred to as branching units.

In a preferred embodiment, the anionic polysaccharide (B) comprises about 0.001 mol% to 5 mol-% branching units, more preferably 0.01 mol% to 3 mol-%, based on all monomers forming anionic polysaccharide (B), based on the total number of repeating units of the anionic polysaccharide (B).

In a preferred embodiment, the anionic polysaccharide (B) is a polypyranose, preferably a polyaldopyranose, optionally cross-linked. The pyranose monomers may be connected e.g. via 1-4 alpha linkages, 1-4 beta linkages, 1-3 alpha linkages and 1-3 beta linkages, respectively.

In another preferred embodiment, the anionic polysaccharide (B) is a polyglucose, optionally substituted with alkoxy, carboxyl, sulfate or phosphate groups, preferably -O-C₁-C₄-alkyl, -C₁-C₄-CO₂H, -OSO₃H, -C₁-C₄-OSO₃H, -OPO₃H₂ or -C₁-C₄-OPO₃H₂.

The glucose monomers may be connected e.g. via 1-4 alpha linkages and 1-4 beta linkages, respectively. Glucose monomers connected via 1-4 alpha linkages to one another form cellulose. Glucose monomers connected via 1-4 beta linkages to one another form amylose.

The anionic polysaccharide (B) may be anionically derivatized at one or more positions of the saccharide moieties (repeating units). When the repeating unit is a glucose moiety, it is preferably anionically derivatized at position 2, 3 and/or 5. In this regard, "anionically derivatized" means that an anionic functional group has been introduced at the respective position, i.e. covalently linked thereto.

Preferably, the anionic polysaccharide (B) contains repeating units according to the formula

[C₆H₇O₂ (OH)ₓ (OR)_{y}]ₙ

wherein
x denotes the degree of free hydroxyl groups and is preferably within the range of from 1.5 to 2.8, more preferably 1.7 to 2.6, still more preferably 1.9 to 2.4;
y denotes the substitution degree and is preferably within the range of from 0.2 to 1.5, more preferably between 0.4 to 1.3, still more preferably 0.6 to 1.1; and
n denotes the average number of repeating units of the polymer, which is preferably within the range of from 100 to 15000, more preferably 200 to 13000 and still more preferably 500 to 10000.

Preferably, x + y is within the range of from 2.0 to 4.0, more preferably 2.5 to 3.5, still more preferably about 3.0.

Each R is preferably independently selected from the group consisting of -C₁-C₄-alkoxy (-O-C₁-C₄-alkyl) or -C₁-C₄-carboxyl, sulfate or phosphate or acceptable salts thereof.

The weight average molecular weight of the anionic polysaccharide (B) is preferably within the range of from 10,000 to 500,000 g mol⁻¹, more preferably 13,000 to 400,000 g mol⁻¹, still more preferably 17,000 to 300,000 g mol⁻¹.

The viscosity of the anionic polysaccharide (B) in 1 wt-% aqueous solution is preferably within the range of from 5 and 20 mPa s, more preferably 8 and 17 mPa s and still more preferably 10 and 15 mPa s.

The viscosity of the anionic polysaccharide (B) in 2 wt-% aqueous solution is preferably within the range of from 15 and 50 mPa s, more preferably 18 and 47 mPa s and still more preferably 20 and 45 mPa s.

The viscosity of the anionic polysaccharide (B) in 4 wt-% aqueous solution is preferably within the range of from 150 and 550 mPa s, more preferably 180 and 520 mPa s and still more preferably 200 and 500 mPa s.

Preferably, at least some of the anionic functional groups contained in the anionic polysaccharide (B) are present in neutralized form, i.e. they are not present in their protonated forms, but are salts with salt-forming cations instead. Suitable salt-forming cations include alkali metal, ammonium, substituted ammonium and amines. More preferably, at least some of the anionic functional groups, e.g. carboxylate and/or sulfonate anions, are salts of sodium or potassium cations.

This percentage of neutralized anionic functional groups, based on the total amount of anionic functional groups, is referred to herein as the "degree of neutralization." In a preferred embodiment, the degree of neutralization is within the range of 35±30%, more preferably 35±25%, still more preferably 35±20%, yet more preferably 35±15%, most preferably 35±10%, and in particular 35±5%. In another preferred embodiment, the degree of neutralization is in the range of 65±30%, more preferably 65±25%, still more preferably 65±20%, yet more preferably 65±15%, most preferably 65±10%, and in particular 65±5%.

The content of anionic polysaccharide (B) ranges preferably from 0.1 wt-% to 95 wt-%, more preferably from 1.0 wt-% to 80 wt-%, still more preferably from 2.0 wt-% to 50 wt-%, and most preferably from 5 wt-% to 30% wt-%, and in particular 9 wt-% to 21 wt-%, based on the total weight of the pharmaceutical dosage form.

In a preferred embodiment, the content of anionic polysaccharide (B) amounts to 0.5 to 35 wt.-%, more preferably 1.0 to 30 wt.-%, still more preferably 2.0 to 32.5 wt.-%, yet more preferably 3.0 to 30 wt.-% and most preferably 4.0 to 27.5 wt.-% and in particular 5.0 to 25 wt.-%, based on the total weight of the pharmaceutical dosage form.

In a preferred embodiment, the content of anionic polysaccharide (B) is within the range of 10±9%, more preferably 10±8%, still more preferably 10±7%, yet more preferably 10±6%, most preferably 10±5%, and in particular 10±2.5%, based on the total weight of the pharmaceutical dosage form.

In still another preferred embodiment, the content of anionic polysaccharide (B) is within the range of 15±14%, more preferably 15±12.5%, still more preferably 15±10%, yet more preferably 15±7.5%, most preferably 15±5%, and in particular 15±2.5%, based on the total weight of the pharmaceutical dosage form.

In still another preferred embodiment, the content of anionic polysaccharide (B) is within the range of 20±15%, more preferably 20±12.5%, still more preferably 20±10%, yet more preferably 20±7.5%, most preferably 20±5%, and in particular 20±2.5%, based on the total weight of the pharmaceutical dosage form.

In a preferred embodiment, the pK_{A} of the anionic polysaccharide (B) is 6.0±2.0, more preferably 6.0±1.5, even more preferably 6.0±1.0, and most preferably 6.0±0.5. In another preferred embodiment, the pK_{A} of the anionic polysaccharide (B) is 7.0±2.0, more preferably 7.0±1.5, even more preferably 7.0±1.0, and most preferably 7.0±0.5. In still another preferred embodiment, the pK_{A} of the anionic polysaccharide (B) is 8.0±2.0, more preferably 8.0±1.5, even more preferably 8.0±1.0, and most preferably 8.0±0.5.

In a preferred embodiment, the pH (in 1 wt% aqueous dispersion) of the anionic polysaccharide (B) is 3.0±3.0, more preferably 3.0±2.0, even more preferably 3.0±1.5, and most preferably 3.0±1.0.

In a preferred embodiment, the pH (in 1 wt% aqueous dispersion) of the anionic polysaccharide (B) is 4.5±3.0, more preferably 4.5±2.0, even more preferably 4.5±1.5, and most preferably 4.5±1.0.

In another preferred embodiment, the pH (in 1 wt% aqueous dispersion) of the anionic polysaccharide (B) is 6.0±3.0, more preferably 6.0±2.0, even more preferably 6.0±1.5, and most preferably 6.0±1.0.

In a preferred embodiment, the anionic polysaccharide (B) preferably exhibits a viscosity of 2,000 to 15,000 mPa s (cp), more preferably 3,000 to 12,000 mPa s, still more preferably 4,000 to 11,000 mPa s, measured by means of a Brookfield viscometer (RVF, 20 rpm) in a 0.5 wt.-% aqueous solution at pH 7.5 and 25°C.

In a preferred embodiment, anionic polysaccharide (B) is homogeneously distributed in the pharmaceutical dosage form according to the invention. Preferably, the pharmacologically active ingredient (A) and anionic polysaccharide (B) are intimately homogeneously distributed in the pharmaceutical dosage form so that the pharmaceutical dosage form does not contain any segments where either pharmacologically active ingredient (A) is present in the absence of anionic polysaccharide (B) or where anionic polysaccharide (B) is present in the absence of pharmacologically active ingredient (A).

When the pharmaceutical dosage form is film coated, the anionic polysaccharide (B) is preferably homogeneously distributed in the core of the pharmaceutical dosage form, i.e. the film coating preferably does not contain anionic polysaccharide (B). Nonetheless, the film coating as such may of course contain one or more polymers, which however, preferably differ from the anionic polysaccharide (B) contained in the core.

The pharmaceutical dosage form according to the invention contains a polyalkylene oxide (C). The active ingredient (A) is embedded into a controlled-release matrix comprising said anionic polysaccharide (B).

Preferably, the polyalkylene oxide (C) is selected from polymethylene oxide, polyethylene oxide and polypropylene oxide, or copolymers thereof.

In a preferred embodiment, the polyalkylene oxide (C) has a weight average molecular weight (M_{w}) or viscosity average molecular weight (M_{η}) of at least 200,000 or at least 500,000 g/mol, preferably at least 1,000,000 g/mol or at least 2,500,000 g/mol, more preferably in the range of about 1,000,000 g/mol to about 15,000,000 g/mol, and most preferably in the range of about 5,000,000 g/mol to about 10,000,000 g/mol. Suitable methods to determine M_{w} and M_{η} are known to a person skilled in the art. M_{η} is preferably determined by rheological measurements, whereas M_{w} can be determined by gel permeation chromatography (GPC).

Preferably, the content of the polyalkylene oxide (C) is within the range of from 20 to 99 wt.-%, more preferably 25 to 95 wt.-%, still more preferably 30 to 90 wt.-%, yet more preferably 30 to 85 wt.-%, most preferably 30 to 80 wt.-% and in particular 30 to 75 wt.-% or 45 to 70 wt.-%, based on the total weight of the pharmaceutical dosage form. In a preferred embodiment, the content of the polyalkylene oxide is at least 20 wt.-%, more preferably at least 25 wt.-%, still more preferably at least 30 wt.-%, yet more preferably at least 35 wt.-% and in particular at least 40 wt.-%.

In a preferred embodiment, the overall content of polyalkylene oxide (C) is within the range of 25±20 wt.-%, more preferably 25±15 wt.-%, most preferably 25±10 wt.-%, and in particular 25±5 wt.-%. In another preferred embodiment, the overall content of polyalkylene oxide (C) is within the range of 35±20 wt.-%, more preferably 35±15 wt.-%, most preferably 35±10 wt.-%, and in particular 35±5 wt.-%. In still another preferred embodiment, the overall content of polyalkylene oxide (C) is within the range of 45±20 wt.-%, more preferably 45±15 wt.-%, most preferably 45±10 wt.-%, and in particular 45±5 wt.-%. In yet another preferred embodiment, the overall content of polyalkylene oxide (C) is within the range of 55±20 wt.-%, more preferably 55±15 wt.-%, most preferably 55±10 wt.-%, and in particular 55±5 wt.-%. In a further preferred embodiment, the overall content of polyalkylene oxide (C) is within the range of 65±20 wt.%, more preferably 65±15 wt.-%, most preferably 65±10 wt.-%, and in particular 65±5 wt.-%. In still a further a preferred embodiment, the overall content of polyalkylene oxide (C) is within the range of 75±20 wt.-%, more preferably 75±15 wt.-%, most preferably 75±10 wt.-%, and in particular 75±5 wt.-%. In a still further a preferred embodiment, the overall content of polyalkylene oxide (C) is within the range of 80±15 wt.-%, more preferably 80±10 wt.-%, and most preferably 80±5 wt.-%.

Polyalkylene oxide (C) may comprise a single polyalkylene oxide having a particular average molecular weight, or a mixture (blend) of different polymers, such as two, three, four or five polymers, e.g., polymers of the same chemical nature but different average molecular weight, polymers of different chemical nature but same average molecular weight, or polymers of different chemical nature as well as different molecular weight.

For the purpose of the specification, a polyalkylene glycol has a molecular weight of up to 20,000 g/mol whereas a polyalkylene oxide has a molecular weight of more than 20,000 g/mol. In a preferred embodiment, the weight average over all molecular weights of all polyalkylene oxides that are contained in the pharmaceutical dosage form is at least 200,000 g/mol. Thus, polyalkylene glycols, if any, are preferably not taken into consideration when determining the weight average molecular weight of polyalkylene oxide (C).

In a preferred embodiment, polyalkylene oxide (C) is homogeneously distributed in the pharmaceutical dosage form according to the invention. Preferably, the pharmacologically active ingredient (A) and polyalkylene oxide (C) are intimately homogeneously distributed in the pharmaceutical dosage form so that the pharmaceutical dosage form does not contain any segments where either pharmacologically active ingredient (A) is present in the absence of polyalkylene oxide (C) or where polyalkylene oxide (C) is present in the absence of pharmacologically active ingredient (A).

When the pharmaceutical dosage form is film coated, the polyalkylene oxide (C) is preferably homogeneously distributed in the core of the pharmaceutical dosage form, i.e. the film coating preferably does not contain polyalkylene oxide (C). Nonetheless, the film coating as such may of course contain one or more polymers, which however, preferably differ from the polyalkylene oxide (C) contained in the core.

The polyalkylene oxide (C) may be combined with one or more different polymers selected from the group consisting of polyalkylene oxide, preferably polymethylene oxide, polyethylene oxide, polypropylene oxide; polyethylene, polypropylene, polyvinyl chloride, polycarbonate, polystyrene, polyvinylpyrrolidone, poly(alk)acrylate, poly(hydroxy fatty acids), such as for example poly(3-hydroxybutyrate-co-3-hydroxyvalerate) (Biopol^{®}), poly(hydroxyvaleric acid); polycaprolactone, polyvinyl alcohol, polyesteramide, polyethylene succinate, polylactone, polyglycolide, polyurethane, polyamide, polylactide, polyacetal (for example polysaccharides optionally with modified side chains), polylactide/glycolide, polylactone, polyglycolide, polyorthoester, polyanhydride, block polymers of polyethylene glycol and polybutylene terephthalate (Polyactive^{®}), polyanhydride (Polifeprosan), copolymers thereof, block-copolymers thereof, and mixtures of at least two of the stated polymers, or other polymers with the above characteristics.

Preferably, the molecular weight dispersity M_{w}/Mₙ of polyalkylene oxide (C) is within the range of 2.5±2.0, more preferably 2.5±1.5, still more preferably 2.5±1.0, yet more preferably 2.5±0.8, most preferably 2.5±0.6, and in particular 2.5±0.4.

The polyalkylene oxide (C) preferably has a viscosity at 25°C of 30 to 17,600 cP, more preferably 55 to 17,600 cP, still more preferably 600 to 17,600 cP and most preferably 4,500 to 17,600 cP, measured in a 5 wt.-% aqueous solution using a model RVF Brookfield viscosimeter (spindle no. 2 / rotational speed 2 rpm); of 400 to 4,000 cP, more preferably 400 to 800 cP or 2,000 to 4,000 cP, measured on a 2 wt.-% aqueous solution using the stated viscosimeter (spindle no. 1 or 3 / rotational speed 10 rpm); or of 1,650 to 10,000 cP, more preferably 1,650 to 5,500 cP, 5,500 to 7,500 cP or 7,500 to 10,000 cP, measured on a 1 wt.-% aqueous solution using the stated viscosimeter (spindle no. 2 / rotational speed 2 rpm).

In a preferred embodiment, the relative weight ratio of polyalkylene oxide (C) to anionic polysaccharide (B) is within the range of from 10:1 to 1:1, more preferably 9:1 to 1.25:1, still more preferably 8:1 to 1.5:1, yet more preferably 7:1 to 1.75:1, most preferably 6.5:1 to 2:1 and in particular 6:1 to 2.5:1.

In another preferred embodiment the relative weight ratio of polyalkylene oxide (C) and anionic polysaccharide (B) is within the range of from 20:1 to 1:20, more preferably 10:1 to 1:10, still more preferably 7:1 to 1:5, yet more preferably 5:1 to 1:1, most preferably 4:1 to 1,5:1 and in particular 3:1 to 2:1. In a preferred embodiment, the relative weight ratio of polyalkylene oxide (C) and anionic polysaccharide (B) is within the range of from 10:1 to 5:1, more preferably 8:1 to 5:1, most preferably 7:1 to 5:1.

Preferably, the relative weight ratio of the polyalkylene oxide (C) to the pharmacologically active ingredient (A) is at least 0.5:1, more preferably at least 1:1, at least 2:1, at least 3:1, at least 4:1, at least 5:1, at least 6:1, at least 7:1, at least 8:1 or at least 9:1; still more preferably at least 10:1 or at least 15:1, yet more preferably at least 20:1, most preferably at least 30:1 1 and in particular at least 40:1. In a preferred embodiment, the relative weight ratio of the polyalkylene oxide (C) to the pharmacologically active ingredient (A) is within the range of from 3:1 to 50:1, more preferably 3:1 to 40:1 and in particular 3:1 to 30:1.

Besides the pharmacologically active ingredient (A), the anionic polysaccharide (B) and the polyalkylene oxide (C) the pharmaceutical dosage form according to the invention may contain further ingredients, e.g. one or more conventional pharmaceutical excipient(s), e.g. fillers, glidants, binding agents, granulating agents, anti-caking agents, lubricants, flavours, dyes, and/or preservatives.

Preferably, the pharmaceutical dosage form further comprises a plasticizer. The plasticizer improves the processability of the polyalkylene oxide (C) and optionally, also of the anionic polysaccharide (B). A preferred plasticizer is polyalkylene glycol, like polyethylene glycol, triacetin, fatty acids, fatty acid esters, waxes and/or microcrystalline waxes. Particularly preferred plasticizers are polyethylene glycols, such as PEG 6000.

Preferably, the content of the plasticizer is within the range of from 0.1 to 25 wt.-%, more preferably 0.5 to 22.5 wt.-%, still more preferably 1.0 to 20 wt.-%, yet more preferably 2.5 to 17.5 wt.-%, most preferably 5.0 to 15 wt.-% and in particular 7.5 to 12.5 wt.-%, based on the total weight of the pharmaceutical dosage form.

In a preferred embodiment, the plasticizer is a polyalkylene glycol having a content within the range of 10±8 wt.-%, more preferably 10±6 wt.-%, still more preferably 10±5 wt.-%, yet more preferably 10±4 wt.-%, most preferably 10±3 wt.-%, and in particular 10±2 wt.-%, based on the total weight of the pharmaceutical dosage form.

In another preferred embodiment, the plasticizer is a polyalkylene glycol having a content within the range of 15±8 wt.-%, more preferably 15±6 wt.-%, still more preferably 15±5 wt.-%, yet more preferably 15±4 wt.-%, most preferably 15±3 wt.-%, and in particular 15±2 wt.-%, based on the total weight of the pharmaceutical dosage form.

In a preferred embodiment, the relative weight ratio of the polyalkylene oxide (C) to the polyalkylene glycol is within the range of 1.5-7.0 : 1, more preferably 2.0-6.5 : 1, still more preferably 2.5-6.0: 1, yet more preferably 3.0-5.5 : 1, most preferably 3.5-5.0: 1, and in particular 3.7-4,7 : 1. This ratio satisfies the requirements of relative high polyalkylene oxide (C) content and good extrudability.

When manufacturing the dosage forms from slices that are obtained by cutting the extrudate strand, the weight of the slices determines the weight of the resulting dosage form. Pronounced variation in weight of these slices results in an accordant weight deviation of dosage forms from the target weight. The weight variation of slices depends strongly on the surface properties of the extrudate strand. A strand with a thoroughly smooth surface allows the generation of slices exhibiting a low weight variation. In contrast, a wavy or shark skinning strand results in slices exhibiting a higher weight variation thereby increasing the number of rejects. It has been surprisingly found that the surface properties of the extrudate strand can be triggered by the polyalkylene oxide : polyalkylene glycol weight ratio.

Preferably, the pharmaceutical dosage form further comprises an anti-oxidant. Suitable oxidants include ascorbic acid, butylated hydroxyanisole (BHA), butylated hydroxytoluene (BHT), salts of ascorbic acid, monothioglycerol, phosphorous acid, vitamin C, vitamin E and the derivatives thereof, coniferyl benzoate, nordihydroguajaretic acid, gallus acid esters, sodium bisulfite, particularly preferably butylhydroxytoluene or butylhydroxyanisole and α-tocopherol. The antioxidant is preferably used in quantities of 0.01 to 10 wt.-%, preferably of 0.03 to 5 wt.-%, relative to the total weight of the pharmaceutical dosage form.

In a preferred embodiment, the pharmaceutical dosage form further comprises an acid, preferably citric acid. The amount of acid is preferably in the range of 0.01 to about 20 wt-%, more preferably in the range of 0.02 to about 10 wt-%, and most preferably in the range of 0.05 to about 5 wt-%.

In a preferred embodiment, the pharmaceutical dosage form contains a natural, semisynthetic or synthetic wax. Waxes with a softening point of at least 50 °C, more preferably 60 °C are preferred. Carnauba wax and beeswax are particularly preferred, especially carnauba wax.

In a preferred embodiment, the pharmaceutical dosage form further comprises another polymer which is preferably selected from cellulose esters and cellulose ethers, in particular hydroxypropyl methylcellulose (HPMC). The amount of the further polymer, preferably hydroxypropyl methylcellulose, preferably ranges from 0.1 wt-% to about 30 wt-%, more preferably in the range of 1.0 wt-% to about 20 wt-%, and most preferably in the range of 2.0 wt-% to about 15 wt-%.

In another preferred embodiment, the pharmaceutical dosage form according to the invention does not contain any further polymer besides the anionic polysaccharide (B), the polyalkylene oxide (C) and optionally, the polyethylene glycol.

The pharmaceutical dosage form according to the invention is preferably an oral dosage form, particularly a tablet. It is also possible, however, to administer the pharmaceutical dosage form via different routes and thus, the pharmaceutical dosage form may alternatively be adapted for buccal, lingual, rectal or vaginal administration. Implants are also possible. Preferably, the pharmaceutical dosage form is monolithic. Preferably, the pharmaceutical dosage form is neither in film form, nor multi-particulate.

In a preferred embodiment, the pharmaceutical dosage form according to the invention is a round tablet. Tablets of this embodiment preferably have a diameter in the range of about 1 mm to about 30 mm, in particular in the range of about 2 mm to about 25 mm, more in particular about 5 mm to about 23 mm, even more in particular about 7 mm to about 13 mm; and a thickness in the range of about 1.0 mm to about 12 mm, in particular in the range of about 2.0 mm to about 10 mm, even more in particular from 3.0 mm to about 9.0 mm, even further in particular from about 4.0 mm to about 8.0 mm.

In another preferred embodiment, the pharmaceutical dosage form according to the invention is an oblong tablet. Tablets of this embodiment preferably have a lengthwise extension (longitudinal extension) of about 1 mm to about 30 mm, in particular in the range of about 2 mm to about 25 mm, more in particular about 5 mm to about 23 mm, even more in particular about 7 mm to about 20 mm; and a thickness in the range of about 1.0 mm to about 12 mm, in particular in the range of about 2.0 mm to about 10 mm, even more in particular from 3.0 mm to about 9.0 mm, even further in particular from about 4.0 mm to about 8.0 mm.

The pharmaceutical dosage form according to the invention has preferably a weight in the range of 0.01 to 1.5 g, more preferably in the range of 0.05 to 1.2 g, still more preferably in the range of 0.1 g to 1.0 g, yet more preferably in the range of 0.2 g to 0.9 g, and most preferably in the range of 0.25 g to 0.8 g.

The pharmaceutical dosage form according to the invention is preferably prepared by thermoforming, although also other methods of thermoforming may be used in order to manufacture the pharmaceutical dosage form according to the invention such as press-molding at elevated temperature or heating of tablets that were manufactured by conventional compression in a first step and then heated above the softening temperature of the polymer in the tablet in a second step to form hard tablets. In this regards, thermoforming means the forming or molding of a mass after the application of heat. In a preferred embodiment, the pharmaceutical dosage form is thermoformed by hot-melt extrusion.

In a preferred embodiment, the pharmaceutical dosage form is prepared by hot melt-extrusion, preferably by means of a twin-screw-extruder. Melt extrusion preferably provides a melt-extruded strand that is preferably cut into monoliths, which are then compressed and formed into tablets. In this regard, the term "tablets" is preferably not to be understood as dosage forms being made by compression of powder or granules (compressi) but rather, as shaped extrudates. Preferably, compression is achieved by means of a die and a punch, preferably from a monolithic mass obtained by melt extrusion. If obtained via melt extrusion, the compressing step is preferably carried out with a monolithic mass exhibiting ambient temperature, that is, a temperature in the range from 20 to 25° C. The strands obtained by way of extrusion can either be subjected to the compression step as such or can be cut prior to the compression step. This cutting can be performed by usual techniques, for example using rotating knives or compressed air. Alternatively, the shaping can take place as described in EP-A 240 906 by the extrudate being passed between two counter-rotating calender rolls and being shaped directly to tablets. It is of course also possible to subject the extruded strands to the compression step or to the cutting step when still warm, that is more or less immediately after the extrusion step. The extrusion is preferably carried out by means of a twin-screw extruder.

The pharmaceutical dosage form of the invention can optionally be provided, partially or completely, with a conventional coating. The dosage forms of the present invention are preferably film coated with conventional film coating compositions.

Suitable coating materials are commercially available, e.g. under the trademarks Opadry^{®} and Eudragit^{®}.

Examples of suitable materials include cellulose esters and cellulose ethers, such as methyl-cellulose (MC), hydroxypropylmethylcellulose (HPMC), hydroxypropylcellulose (HPC), hydroxyethylcellulose (HEC), sodium carboxymethylcellulose (Na-CMC), ethylcellulose (EC), cellulose acetate phthalate (CAP), hydroxypropylmethylcellulose phthalate (HPMCP); poly(meth)acrylates, such as aminoalkylmethacrylate copolymers, ethylacrylate methylmethacrylate copolymers, methacrylic acid methylmethacrylate copolymers, methacrylic acid methylmethacrylate copolymers; vinyl polymers, such as polyvinylpyrrolidone, polyvinyl-acetatephthalate, polyvinyl alcohol, polyvinylacetate; and natural film formers, such as shellack.

In a particularly preferred embodiment, the coating is water-soluble. In a preferred embodiment, the coating is based on polyvinyl alcohol, such as polyvinyl alcohol-part. Hydrolyzed, and may additionally contain polyethylene glycol, such as macrogol 3350, and/or pigments. In another preferred embodiment, the coating is based on hydroxypropylmethyl-cellulose, preferably hypromellose type 2910 having a viscosity of 3 to 15 mPas.

The coating can be resistant to gastric juices and dissolve as a function of the pH value of the release environment. By means of this coating, it is possible to ensure that the pharmaceutical dosage form according to the invention passes through the stomach undissolved and the active ingredient is only released in the intestines. The coating which is resistant to gastric juices preferably dissolves at a pH value of between 5 and 7.5. Corresponding materials and methods for the delayed release of active ingredients and for the application of coatings which are resistant to gastric juices are known to the person skilled in the art, for example from "Coated Pharmaceutical dosage forms - Fundamentals, Manufacturing Techniques, Biopharmaceutical Aspects, Test Methods and Raw Materials" by Kurt H. Bauer, K. Lehmann, Hermann P. Osterwald, Rothgang, Gerhart, 1st edition, 1998, Medpharm Scientific Publishers.

The coating can also be applied e.g. to improve the aesthetic impression and/or the taste of the dosage forms and the ease with which they can be swallowed. Coating the dosage forms of the present invention can also serve other purposes, e.g. improving stability and shelf-life. Suitable coating formulations comprise a film forming polymer such as, for example, polyvinyl alcohol or hydroxypropyl methylcellulose, e.g. hypromellose, a plasticizer such as, for example, a glycol, e.g. propylene glycol or polyethylene glycol, an opacifier, such as, for example, titanium dioxide, and a film smoothener, such as, for example, talc. Suitable coating solvents are water as well as organic solvents. Examples of organic solvents are alcohols, e.g. ethanol or isopropanol, ketones, e.g. acetone, or halogenated hydrocarbons, e.g. methylene chloride. Optionally, the coating can contain a therapeutically effective amount of one or more active ingredients to provide for an immediate release of said active ingredient (A) and thus for an immediate relief of the symptoms treated by said active ingredient (A). Coated dosage forms of the present invention are preferably prepared by first making the cores and subsequently coating said cores using conventional techniques, such as coating in a coating pan.

According to the invention, the active ingredient (A) is embedded in a controlled-release matrix comprising anionic polysaccharide (B) and polyalkylene oxide (C).

Controlled release of an active ingredient from an oral dosage form is known to a person skilled in the art. For the purpose of the specification, controlled release encompasses delayed release, retarded release, sustained release, prolonged release, and the like.

Controlled or prolonged release is understood according to the invention preferably to mean a release profile in which the pharmacologically active ingredient (A) is released over a relatively long period with reduced intake frequency with the purpose of extended therapeutic action. Preferably, the meaning of the term "prolonged release" is in accordance with the European guideline on the nomenclature of the release profile of pharmaceutical dosage forms (CHMP). This is achieved in particular with peroral administration. The expression "at least partially delayed or prolonged release" covers according to the invention any pharmaceutical dosage forms which ensure modified release of the opioids (A) contained therein. The pharmaceutical dosage forms preferably comprise coated or uncoated pharmaceutical dosage forms, which are produced with specific auxiliary substances, by particular processes or by a combination of the two possible options in order purposefully to change the release rate or location of release.

In the case of the pharmaceutical dosage forms according to the invention, the release time profile of a controlled release form may be modified e.g. as follows: extended release, repeat action release, prolonged release and sustained release.

For the purpose of the specification "controlled release" preferably means a product in which the release of active ingredient over time is controlled by the type and composition of the formulation. For the purpose of the specification "extended release" preferably means a product in which the release of active ingredient is delayed for a finite lag time, after which release is unhindered. For the purpose of the specification "repeat action release" preferably means a product in which a first portion of active ingredient is released initially, followed by at least one further portion of active ingredient being released subsequently. For the purpose of the specification "prolonged release" preferably means a product in which the rate of release of active ingredient from the formulation after administration has been reduced over time, in order to maintain therapeutic activity, to reduce toxic effects, or for some other therapeutic purpose. For the purpose of the specification "sustained release" preferably means a way of formulating a medicine so that it is released into the body steadily, over a long period of time, thus reducing the dosing frequency. For further details, reference may be made, for example, to K.H. Bauer, Lehrbuch der Pharmazeutischen Technologie, 6th edition, WVG Stuttgart, 1999; and Eur. Ph.

Preferably, under physiological conditions the pharmaceutical dosage form according to the invention has released after 30 minutes 0.1 to 75%, after 240 minutes 0.5 to 95%, after 480 minutes 1.0 to 100% and after 720 minutes 2.5 to 100% of the pharmacologically active ingredient (A). Further preferred release profiles R₁ to R₆ are summarized in the table here below [all data in wt.-% of released pharmacologically active ingredient (A)]:

| time | R₁ | R₂ | R₃ | R₄ | R₅ | R₆ |
|---|---|---|---|---|---|---|
| 60 min | 0-30 | 0-50 | 0-50 | 15-25 | 20-30 | 20-50 |
| 120 min | 0-40 | 0-75 | 0-75 | 25-40 | 35-50 | 40-75 |
| 240 min | 3-55 | 3-95 | 10-95 | 40-70 | 55-75 | 60-95 |
| 480 min | 10-65 | 10-100 | 35-100 | 60-90 | 80-95 | 80-100 |
| 720 min | 20-75 | 20-100 | 55-100 | 70-100 | 90-100 | 90-100 |
| 960 min | 30-88 | 30-100 | 70-100 | >80 | 95-100 | |
| 1440 min | 50-100 | 50-100 | >90 | | | |
| 2160 min | >80 | >80 | | | | |

Further preferred release profiles R₇ to R₁₂ are summarized in the table here below [all data in wt.-% of released pharmacologically active ingredient (A)]:

| time | R₇ | R₈ | R₉ | R₁₀ | R₁₁ | R₁₂ |
|---|---|---|---|---|---|---|
| 30 min | 17.5±7.5 | 17.5±6.5 | 17.5±5.5 | 17.5±4.5 | 17.5±3.5 | 17.5±2.5 |
| 60 min | 27.0±8.0 | 27.0±7.0 | 27.0±6.0 | 27.0±5.0 | 27.0±4.0 | 27.0±3.0 |
| 120 min | 41.5±9.5 | 41.5±8.5 | 41.5±7.5 | 41.5±6.5 | 41.5±5.5 | 41.5±4.5 |
| 240 min | 64.5±12.5 | 64.5±11.5 | 64.5±10.5 | 64.5±9.5 | 64.5±8.5 | 64.5±7.5 |
| 480 min | 88.0±12.0 | 88.0±11.0 | 88.0±10.0 | 88.0±9.0 | 88.0±8.0 | 88.0±7.0 |
| 720 min | 96.0±9.0 | 96.0±8.0 | 96.0±7.0 | 96.0±6.0 | 96.0±5.0 | 96.0±4.0 |
| 840 min | 97.5±7.5 | 97.5±6.5 | 97.5±5.5 | 97.5±4.5 | 97.5±3.5 | 97.5±2.5 |

Preferably, the release profile of the pharmaceutical dosage form according to the present invention is stable upon storage, preferably upon storage at elevated temperature, e.g. 40°C, for 3 months in sealed containers. In this regard "stable" means that when comparing the initial release profile with the release profile after storage, at any given time point the release profiles deviate from one another by not more than 20%, more preferably not more than 15%, still more preferably not more than 10%, yet more preferably not more than 7.5%, most preferably not more than 5.0% and in particular not more than 2.5%.

Preferably, under *in vitro* conditions the pharmaceutical dosage form has released after 0.5 h 1.0 to 35 wt.-%, after 1 h 5.0 to 45 wt.-%, after 2 h 10 to 60 wt.-%, after 4 h at least 15 wt.-%, after 6 h at least 20 wt.-%, after 8 h at least 25 wt.-% and after 12 h at least 30 wt.-% of the pharmacologically active ingredient (A) that was originally contained in the pharmaceutical dosage form.

Suitable in vitro conditions are known to the skilled artisan. In this regard it can be referred to, e.g., the Eur. Ph. Preferably, the release profile is measured under the following conditions: Paddle apparatus equipped with sinker, 75 rpm, 37±5 °C, 600 mL simulated intestinal fluid pH 6.8 (phosphate buffer) or pH 4.5. In a preferred embodiment, the rotational speed of the paddle is increased to 100 rpm.

In a preferred embodiment, the pharmaceutical dosage form according to the invention is adapted for administration once daily. In another preferred embodiment, the pharmaceutical dosage form according to the invention is adapted for administration twice daily. In still another preferred embodiment, the pharmaceutical dosage form according to the invention is adapted for administration thrice daily.

For the purpose of the specification, "twice daily" means equal or nearly equal time intervals, i.e., about every 12 hours, or different time intervals, e.g., 8 and 16 hours or 10 and 14 hours, between the individual administrations.

For the purpose of the specification, "thrice daily" means equal or nearly equal time intervals, i.e., about every 8 hours, or different time intervals, e.g., 6, 6 and 12 hours; or 7, 7 and 10 hours, between the individual administrations.

Preferably the pharmaceutical dosage form according to the invention releases after 5 h at most 99%, more preferably at most 90%, still more preferably at most 75%, and most preferably at most 60% of the active ingredient (A).

The anionic polysaccharide (B) is preferably hydrophilic, meaning that a matrix comprising anionic polysaccharide (B) and polyalkylene oxide (C) tends to swell upon contact with aqueous fluids following administration, and preferably results in a viscous, drug release regulating gel layer.

In a preferred embodiment, the matrix comprising the anionic polysaccharide (B) and the polyalkylene oxide (C) contains anionic polysaccharide (B) in such a quantity that:
a) under *in vitro* conditions the release of the active ingredient (A) is additionally retarded; and/or
b) upon exposure to water the hydration process of the pharmaceutical dosage form is accelerated; during this process the pharmaceutical dosage form forms a water-containing shell (gel) around a dry core in such a way that during the first 270 minutes after the exposure, the core/gel ratio decreases while the volume of the said dosage form increases not more than 20 %, in particular not more than 10 % of the original volume of the dosage form;
   in each case compared to a thus identical, comparative pharmaceutical dosage form wherein the anionic polysaccharide (B) is substituted with the corresponding amount of hydroxypropyl methyl cellulose (HPMC).

In a particular preferred embodiment,
- the pharmaceutical dosage form is thermoformed, preferably by hot melt-extrusion; and/or
- the pharmaceutical dosage form exhibits a breaking strength of at least 1500 N; and/or
- the pharmaceutical dosage form is adapted for administration once-daily, twice daily or thrice-daily; and/or
- the pharmacologically active ingredients (A) is selected from the group of opioids and opiates; and or
- anionic polysaccharide (B) is obtainable by introducing anionic functional groups, in protonated form or a physiologically acceptable salt thereof, into a polysaccharide, wherein the anionic functional groups are selected from unsaturated carboxylic acids and acid anhydrides, unsaturated sulfonic acids and mixtures thereof; and/or
- the content of anionic polysaccharide (B) ranges from 2.0 wt-% to 50 wt-%; and/or
- the polyalkylene oxide (C) is selected from polymethylene oxide, polyethylene oxide and polypropylene oxide, or copolymers thereof; having a weight average molecular weight (M_{w}) of at least 500,000 g/mol, more preferably within the range of from 1,000,000 g/mol to 10,000,000 g/mol; and/or
- the content of polyalkylene oxide (C) is at least 30 wt-%, based on the total weight of the dosage form.

In a preferred embodiment, the pharmaceutical dosage form according to the invention contains no substances which irritate the nasal passages and/or pharynx, i.e. substances which, when administered via the nasal passages and/or pharynx, bring about a physical reaction which is either so unpleasant for the patient that he/she does not wish to or cannot continue administration, for example burning, or physiologically counteracts taking of the corresponding active ingredient, for example due to increased nasal secretion or sneezing. Further examples of substances which irritate the nasal passages and/or pharynx are those which cause burning, itching, urge to sneeze, increased formation of secretions or a combination of at least two of these stimuli. Corresponding substances and the quantities thereof which are conventionally to be used are known to the person skilled in the art. Some of the substances which irritate the nasal passages and/or pharynx are accordingly based on one or more constituents or one or more plant parts of a hot substance drug. Corresponding hot substance drugs are known per se to the person skilled in the art and are described, for example, in "Pharmazeutische Biologie - Drogen und ihre Inhaltsstoffe" by Prof. Dr. Hildebert Wagner, 2nd., revised edition, Gustav Fischer Verlag, Stuttgart-New York, 1982, pages 82 et seq.. The corresponding description is hereby introduced as a reference and is deemed to be part of the disclosure.

The pharmaceutical dosage form according to the invention furthermore preferably contains no antagonists for the pharmacologically active ingredient (A), preferably no antagonists against psychotropic substances, in particular no antagonists against opioids (A). Antagonists suitable for a given pharmacologically active ingredient (A) are known to the person skilled in the art and may be present as such or in the form of corresponding derivatives, in particular esters or ethers, or in each case in the form of corresponding physiologically acceptable compounds, in particular in the form of the salts or solvates thereof. The pharmaceutical dosage form according to the invention preferably contains no antagonists selected from among the group comprising naloxone, naltrexone, nalmefene, nalide, nalmexone, nalorphine or naluphine, in each case optionally in the form of a corresponding physiologically acceptable compound, in particular in the form of a base, a salt or solvate; and no neuroleptics, for example a compound selected from among the group comprising haloperidol, promethacine, fluphenazine, perphenazine, levomepromazine, thioridazine, perazine, chlorpromazine, chlorprothixine, zuclopenthixol, flupentixol, prothipendyl, zotepine, benperidol, pipamperone, melperone and bromperidol.

The pharmaceutical dosage form according to the invention furthermore preferably contains no emetic. Emetics are known to the person skilled in the art and may be present as such or in the form of corresponding derivatives, in particular esters or ethers, or in each case in the form of corresponding physiologically acceptable compounds, in particular in the form of the salts or solvates thereof. The pharmaceutical dosage form according to the invention preferably contains no emetic based on one or more constituents of ipecacuanha (ipecac) root, for example based on the constituent emetine, as are, for example, described in "Pharmazeutische Biologie - Drogen und ihre Inhaltsstoffe" by Prof. Dr. Hildebert Wagner, 2nd, revised edition, Gustav Fischer Verlag, Stuttgart, New York, 1982. The corresponding literature description is hereby introduced as a reference and is deemed to be part of the disclosure. The pharmaceutical dosage form according to the invention preferably also contains no apomorphine as an emetic.

Finally, the pharmaceutical dosage form according to the invention preferably also contains no bitter substance. Bitter substances and the quantities effective for use may be found in US-2003/0064099 A1, the corresponding disclosure of which should be deemed to be the disclosure of the present application and is hereby introduced as a reference. Examples of bitter substances are aromatic oils, such as peppermint oil, eucalyptus oil, bitter almond oil, menthol, fruit aroma substances, aroma substances from lemons, oranges, limes, grapefruit or mixtures thereof, and/or denatonium benzoate.

The pharmaceutical dosage form according to the invention accordingly preferably contains neither substances which irritate the nasal passages and/or pharynx, nor antagonists for the pharmacologically active ingredient (A), nor emetics, nor bitter substances.

The pharmaceutical dosage form according to the invention has a breaking strength of at least 500 N.

The pharmaceutical dosage form according to the invention is preferably tamper-resistant. Preferably, tamper-resistance is achieved based on the mechanical properties of the pharmaceutical dosage form so that comminution is avoided or at least substantially impeded. According to the invention, the term comminution means the pulverization of the pharmaceutical dosage form using conventional means usually available to an abuser, for example a pestle and mortar, a hammer, a mallet or other conventional means for pulverizing under the action of force. Thus, tamper-resistance preferably means that pulverization of the pharmaceutical dosage form using conventional means is avoided or at least substantially impeded.

Preferably, the mechanical properties of the pharmaceutical dosage form according to the invention, particularly its breaking strength, substantially rely on the presence and spatial distribution of anionic polysaccharide (B) and polyalkylene oxide (C), although their mere presence does typically not suffice in order to achieve said properties. The advantageous mechanical properties of the pharmaceutical dosage form according to the invention may not automatically be achieved by simply processing pharmacologically active ingredient (A), anionic polysaccharide (B), polyalkylene oxide (C), and optionally further excipients by means of conventional methods for the preparation of pharmaceutical dosage forms. In fact, usually suitable apparatuses must be selected for the preparation and critical processing parameters must be adjusted, particularly pressure/force, temperature and time. Thus, even if conventional apparatuses are used, the process protocols usually must be adapted in order to meet the required criteria.

In general, the dosage forms exhibiting the desired properties may be obtained only if, during preparation of the dosage form,
- suitable components
- in suitable amounts
   are exposed to
- a sufficient pressure
- at a sufficient temperature
- for a sufficient period of time.

Thus, regardless of the apparatus used, the process protocols must be adapted in order to meet the required criteria. Therefore, the breaking strength is separable from the composition.

The pharmaceutical dosage form according to the invention has a breaking strength of at least 500 N, preferably at least 600 N, more preferably at least 700 N, still more preferably at least 800 N, yet more preferably at least 1000 N, most preferably at least 1250 N and in particular at least 1500 N.

The "breaking strength" (resistance to crushing) of a pharmaceutical dosage form is known to the skilled person. In this regard it can be referred to, e.g., W.A. Ritschel, Die Tablette, 2. Auflage, Editio Cantor Verlag Aulendorf, 2002; H Liebermann et al., Pharmaceutical dosage forms: Tablets, Vol. 2, Informa Healthcare; 2 edition, 1990; and Encyclopedia of Pharmaceutical Technology, Informa Healthcare; 1 edition.

For the purpose of the specification, the breaking strength is preferably defined as the amount of force that is necessary in order to fracture the pharmaceutical dosage form (= breaking force). Therefore, for the purpose of the specification the pharmaceutical dosage form does preferably not exhibit the desired breaking strength when it breaks, i.e., is fractured into at least two independent parts that are separated from one another. In another preferred embodiment, however, the pharmaceutical dosage form is regarded as being broken if the force decreases by 25% (threshold value) of the highest force measured during the measurement (see below).

The pharmaceutical dosage forms according to the invention are distinguished from conventional pharmaceutical dosage forms in that, due to their breaking strength, they cannot be pulverized by the application of force with conventional means, such as for example a pestle and mortar, a hammer, a mallet or other usual means for pulverization, in particular devices developed for this purpose (tablet crushers). In this regard "pulverization" means crumbling into small particles that would immediately release the pharmacologically active ingredient (A) in a suitable medium. Avoidance of pulverization virtually rules out oral or parenteral, in particular intravenous or nasal abuse.

Conventional tablets typically have a breaking strength well below 200 N in any direction of extension. The breaking strength of conventional round tablets may be estimated according to the following empirical formula: *Breaking Strength* [in N] = *10 x Diameter Of The Tablet* [in mm]. Thus, according to said empirical formula, a round tablet having a breaking strength of at least 300 N would require a diameter of at least 30 mm). Such a tablet, however, could not be swallowed. The above empirical formula preferably does not apply to the pharmaceutical dosage forms of the invention, which are not conventional but rather special.

Further, the actual mean chewing force is about 220 N (cf., e.g., P.A. Proeschel et al., J Dent Res, 2002, 81 (7), 464-468). This means that conventional tablets having a breaking strength well below 200 N may be crushed upon spontaneous chewing, whereas the pharmaceutical dosage forms according to the invention may not.

Still further, when applying a gravitational acceleration of about 9.81 m/s², 500 N correspond to a gravitational force of more than 50 kg, i.e. the pharmaceutical dosage forms according to the invention can preferably withstand a weight of more than 50 kg without being pulverized.

Methods for measuring the breaking strength of a pharmaceutical dosage form are known to the skilled artisan. Suitable devices are commercially available.

For example, the breaking strength (resistance to crushing) can be measured in accordance with the Eur. Ph. 5.0, 2.9.8 or 6.0, 2.09.08 "Resistance to Crushing of Tablets". The test is intended to determine, under defined conditions, the resistance to crushing of tablets, measured by the force needed to disrupt them by crushing. The apparatus consists of 2 jaws facing each other, one of which moves towards the other. The flat surfaces of the jaws are perpendicular to the direction of movement. The crushing surfaces of the jaws are flat and larger than the zone of contact with the tablet. The apparatus is calibrated using a system with a precision of 1 Newton. The tablet is placed between the jaws, taking into account, where applicable, the shape, the break-mark and the inscription; for each measurement the tablet is oriented in the same way with respect to the direction of application of the force (and the direction of extension in which the breaking strength is to be measured). The measurement is carried out on 10 tablets, taking care that all fragments of tablets have been removed before each determination. The result is expressed as the mean, minimum and maximum values of the forces measured, all expressed in Newton.

A similar description of the breaking strength (breaking force) can be found in the USP. The breaking strength can alternatively be measured in accordance with the method described therein where it is stated that the breaking strength is the force required to cause a tablet to fail (i.e., break) in a specific plane. The tablets are generally placed between two platens, one of which moves to apply sufficient force to the tablet to cause fracture. For conventional, round (circular cross-section) tablets, loading occurs across their diameter (sometimes referred to as diametral loading), and fracture occurs in the plane. The breaking force of tablets is commonly called hardness in the pharmaceutical literature; however, the use of this term is misleading. In material science, the term hardness refers to the resistance of a surface to penetration or indentation by a small probe. The term crushing strength is also frequently used to describe the resistance of tablets to the application of a compressive load. Although this term describes the true nature of the test more accurately than does hardness, it implies that tablets are actually crushed during the test, which is often not the case.

Alternatively, the breaking strength (resistance to crushing) can be measured in accordance with WO 2005/ 016313, WO 2005/016314, and WO 2006/082099, which can be regarded as a modification of the method described in the Eur. Ph. The apparatus used for the measurement is preferably a "Zwick Z 2.5" materials tester, Fₘₐₓ = 2.5 kN with a maximum draw of 1150 mm, which should be set up with one column and one spindle, a clearance behind of 100 mm and a test speed adjustable between 0.1 and 800 mm/min together with testControl software. Measurement is performed using a pressure piston with screw-in inserts and a cylinder (diameter 10 mm), a force transducer, Fₘₐₓ. 1 kN, diameter = 8 mm, class 0.5 from 10 N, class 1 from 2 N to ISO 7500-1, with manufacturer's test certificate M according to DIN 55350-18 (Zwick gross force Fₘₐₓ = 1.45 kN) (all apparatus from Zwick GmbH & Co. KG, Ulm, Germany) with Order No BTC-FR 2.5 TH. D09 for the tester, Order No BTC-LC 0050N. P01 for the force transducer, Order No BO 70000 S06 for the centering device.

In a preferred embodiment of the invention, the breaking strength is measured by means of a breaking strength tester e.g. Sotax^{®}, type HT100 or type HT1 (Allschwil, Switzerland). Both, the Sotax^{®} HT100 and the Sotax^{®} HT1 can measure the breaking strength according to two different measurement principles: constant speed (where the test jaw is moved at a constant speed adjustable from 5-200 mm/min) or constant force (where the test jaw increases force linearly adjustable from 5-100 N/sec). In principle, both measurement principles are suitable for measuring the breaking strength of the pharmaceutical dosage form according to the invention. Preferably, the breaking strength is measured at constant speed, preferably at a constant speed of 120 mm/min.

In a preferred embodiment, the pharmaceutical dosage form is regarded as being broken if it is fractured into at least two separate pieces.

The pharmaceutical dosage form according to the invention preferably exhibits mechanical strength over a wide temperature range, in addition to the breaking strength (resistance to crushing) optionally also sufficient hardness, impact resistance, impact elasticity, tensile strength and/or modulus of elasticity, optionally also at low temperatures (e.g. below -24 °C, below -40 °C or in liquid nitrogen), for it to be virtually impossible to pulverize by spontaneous chewing, grinding in a mortar, pounding, etc. Thus, preferably, in direction of extension E₁ the comparatively high breaking strength of the pharmaceutical dosage form according to the invention is maintained even at low or very low temperatures, e.g., when the pharmaceutical dosage form is initially chilled to increase its brittleness, for example to temperatures below-25°C, below -40 °C or even in liquid nitrogen.

The pharmaceutical dosage form according to the invention is characterized by a certain degree of breaking strength. This does not mean that the pharmaceutical dosage form must also exhibit a certain degree of hardness. Hardness and breaking strength are different physical properties. Therefore, the tamper resistance of the pharmaceutical dosage form does not necessarily depend on the hardness of the pharmaceutical dosage form. For instance, due to its breaking strength, impact strength, elasticity modulus and tensile strength, respectively, the pharmaceutical dosage form can preferably be deformed, e.g. plastically, when exerting an external force, for example using a hammer, but cannot be pulverized, i.e., crumbled into a high number of fragments. In other words, the pharmaceutical dosage form according to the invention is characterized by a certain degree of breaking strength, but not necessarily also by a certain degree of form stability.

Therefore, in the meaning of the specification, a pharmaceutical dosage form that is deformed when being exposed to a force in a particular direction of extension but that does not break (plastic deformation or plastic flow) is preferably to be regarded as having the desired breaking strength in said direction of extension.

In a preferred embodiment, the sodiumcarboxymethylstarch as a part of the anionic polysaccharide (B) according to the invention contains not more than 25 % NaCl, preferably not more than 15 % NaCI, more preferably not more than 10 % NaCl and especially preferred not more than 7 % NaCl. The pH value of the sodiumcarboxymethylstarch is preferably between 3.5 and 5.0. Drying the sodiumcarboxymethylstarch at 130 °C for at least 90 minutes results in a loss of weight of preferably not more than 25 %, more preferably not more than 20 %, yet more preferably not more than 15 %, especially preferred not more than 10%.

The carboxymethyl cellulose sodium as a part of the anionic polysaccharide (B) according to the invention contains not more than 25 % sodium, preferably not more than 15 % sodium, more preferably not more than 12 % sodium and especially preferred between 6.5 % and 9.5 % sodium. The pH value of the carboxymethyl cellulose sodium is preferably between 6.5 and 8.5. Drying the carboxymethyl cellulose sodium at 105 °C for at least 180 minutes results in a loss of weight of preferably not more than 25 %, more preferably not more than 20 %, yet more preferably not more than 15 %, especially preferred not more than 10 %.

The carboxymethyl cellulose as a part of the anionic polysaccharide (B) according to the invention has a pH value which is preferably between 6.0 and 8.5. Drying the carboxymethyl cellulose at 130 °C for at least 90 minutes results in a loss of weight of preferably not more than 25 %, more preferably not more than 20 %, yet more preferably not more than 15 %, especially preferred not more than 8 %.

The crosscarmellose as a part of the anionic polysaccharide (B) according to the invention has a pH value which is preferably between 5.0 and 7.0. Drying the Crosscarmellose at 105 °C for at least 300 minutes results in a loss of weight of preferably not more than 25 %, more preferably not more than 20 %, yet more preferably not more than 15 %, especially preferred not more than 8 %.

In a preferred embodiment the invention relates to a tamper-resistant pharmaceutical dosage form having a retarded release profile, especially a tamper-resistant oral dosage form having a retarded release profile, particularly a tamper-resistant tablet having a retarded release profile comprising at least one pharmaceutically active ingredient (A) (pharmacologically active compound) with potential for abuse.

The pharmaceutical dosage form according to the invention may be produced by different processes, the particularly preferred of which are explained in greater detail below. Several suitable processes have already been described in the prior art. In this regard it can be referred to, e.g., WO 2005/ 016313, WO 2005/016314, WO 2005/063214, WO 2005/102286, WO 2006/002883, WO 2006/002884, WO 2006/002886, WO 2006/082097, and WO 2006/082099.

The present invention also relates to pharmaceutical dosage forms that are obtainable by any of the processes described here below.

In general, the process for the production of the pharmaceutical dosage form according to the invention preferably comprises the following steps:
(a) mixing all ingredients;
(b) optionally pre-forming the mixture obtained from step (a), preferably by applying heat and/or force to the mixture obtained from step (a), the quantity of heat supplied preferably not being sufficient to heat the polyalkylene oxide (C) up to its softening point;
(c) hardening the mixture by applying heat and force, and after the process decreasing heat and force, it being possible to supply the heat during and/or before the application of force and the quantity of heat supplied being sufficient to heat the polyalkylene oxide (C) at least up to its softening point;
(d) optionally singulating the hardened mixture;
(e) optionally shaping the pharmaceutical dosage form; and
(f) optionally providing a film coating.

Heat may be supplied directly, e.g. by contact or by means of hot gas such as hot air, or with the assistance of ultrasound; or is indirectly supplied by friction and/or shear. Force may be applied and/or the pharmaceutical dosage form may be shaped for example by direct tabletting or with the assistance of a suitable extruder, particularly by means of a screw extruder equipped with two screws (twin-screw-extruder) or by means of a planetary gear extruder.

The final shape of the pharmaceutical dosage form may either be provided during the hardening of the mixture by applying heat and force (step (c)) or in a subsequent step (step (e)). In both cases, the mixture of all components is preferably in the plastified state, i.e. preferably, shaping is performed at a temperature at least above the softening point of the polyalkylene oxide (C). However, extrusion at lower temperatures, e.g. ambient temperature, is also possible and may be preferred.

Shaping can be performed, e.g., by means of a tabletting press comprising die and punches of appropriate shape.

A particularly preferred process for the manufacture of the pharmaceutical dosage form of the invention involves hot-melt extrusion. In this process, the pharmaceutical dosage form according to the invention is produced by thermoforming with the assistance of an extruder, preferably without there being any observable consequent discoloration of the extrudate.

This process is characterized in that
a) all components are mixed,
b) the resultant mixture is heated in the extruder at least up to the softening point of the polyalkylene oxide (C) and extruded through the outlet orifice of the extruder by application of force,
c) the still plastic extrudate is singulated and formed into the pharmaceutical dosage form
   or
d) the cooled and optionally reheated singulated extrudate is formed into the pharmaceutical dosage form.

Mixing of the components according to process step a) may also proceed in the extruder.

The components may also be mixed in a mixer known to the person skilled in the art. The mixer may, for example, be a roll mixer, shaking mixer, shear mixer or compulsory mixer.

The, preferably molten, mixture which has been heated in the extruder at least up to the softening point of polyalkylene oxide (C) is extruded from the extruder through a die with at least one bore.

The process according to the invention requires the use of suitable extruders, preferably screw extruders. Screw extruders which are equipped with two screws (twin-screw-extruders) are particularly preferred.

The extrusion is preferably performed so that the expansion of the strand due to extrusion is not more than 30%, i.e. that when using a die with a bore having a diameter of e.g. 6 mm, the extruded strand should have a diameter of not more than 8 mm. More preferably, the expansion of the strand is not more than 25%, still more preferably not more than 20%, most preferably not more than 15% and in particular not more than 10%.

Preferably, extrusion is performed in the absence of water, i.e., no water is added. However, traces of water (e.g., caused by atmospheric humidity) may be present.

The extruder preferably comprises at least two temperature zones, with heating of the mixture at least up to the softening point of the polyalkylene oxide (C) proceeding in the first zone, which is downstream from a feed zone and optionally mixing zone. The throughput of the mixture is preferably from 1.0 kg to 15 kg/hour. In a preferred embodiment, the throughput is from 1 to 3.5 kg/hour. In another preferred embodiment, the throughput is from 4 to 15 kg/hour.

In a preferred embodiment, the die head pressure is within the range of from 25 to 100 bar. The die head pressure can be adjusted inter alia by die geometry, temperature profile and extrusion speed.

The die geometry or the geometry of the bores is freely selectable. The die or the bores may accordingly exhibit a round, oblong or oval cross-section, wherein the round cross-section preferably has a diameter of 0.1 mm to 15 mm and the oblong cross-section preferably has a maximum lengthwise extension of 21 mm and a crosswise extension of 10 mm. Preferably, the die or the bores have a round cross-section. The casing of the extruder used according to the invention may be heated or cooled. The corresponding temperature control, i.e. heating or cooling, is so arranged that the mixture to be extruded exhibits at least an average temperature (product temperature) corresponding to the softening temperature of the polyalkylene oxide (C) and does not rise above a temperature at which the pharmacologically active ingredient (A) to be processed may be damaged. Preferably, the temperature of the mixture to be extruded is adjusted to below 180 °C, preferably below 150 °C, but at least to the softening temperature of polyalkylene oxide (C). Typical extrusion temperatures are 120 °C and 130 °C.

In a preferred embodiment, the extruder torque is within the range of from 30 to 95%. Extruder torque can be adjusted inter alia by die geometry, temperature profile and extrusion speed.

After extrusion of the molten mixture and optional cooling of the extruded strand or extruded strands, the extrudates are preferably singulated. This singulation may preferably be performed by cutting up the extrudates by means of revolving or rotating knives, water jet cutters, wires, blades or with the assistance of laser cutters.

Preferably, intermediate or final storage of the optionally singulated extrudate or the final shape of the pharmaceutical dosage form according to the invention is performed under oxygen-free atmosphere which may be achieved, e.g., by means of oxygen-scavengers.

The singulated extrudate may be press-formed into tablets in order to impart the final shape to the pharmaceutical dosage form.

The application of force in the extruder onto the at least plasticized mixture is adjusted by controlling the rotational speed of the conveying device in the extruder and the geometry thereof and by dimensioning the outlet orifice in such a manner that the pressure necessary for extruding the plasticized mixture is built up in the extruder, preferably immediately prior to extrusion. The extrusion parameters which, for each particular composition, are necessary to give rise to a pharmaceutical dosage form with desired mechanical properties, may be established by simple preliminary testing.

For example but not limiting, extrusion may be performed by means of a twin-screw-extruder type ZSE 18 or ZSE27 (Leistritz, Nürnberg, Germany), screw diameters of 18 or 27 mm. Screws having eccentric ends may be used. A heatable die with a round bore having a diameter of 7, 8, or 9 mm may be used. The extrusion parameters may be adjusted e.g. to the following values: rotational speed of the screws: 120 Upm; delivery rate 2 kg/h for a ZSE 18 or 8 kg/h for a ZSE27; product temperature: in front of die 125 °C and behind die 135 °C; and jacket temperature: 110 °C.

Preferably, extrusion is performed by means of twin-screw-extruders or planetary-gear-extruders, twin-screw extruders (co-rotating or contra-rotating) being particularly preferred.

The pharmaceutical dosage form according to the invention is preferably produced by thermoforming with the assistance of an extruder without any observable consequent discoloration of the extrudates.

The process for the preparation of the pharmaceutical dosage form according to the invention is preferably performed continuously. Preferably, the process involves the extrusion of a homogeneous mixture of all components. It is particularly advantageous if the thus obtained intermediate, e.g. the strand obtained by extrusion, exhibits uniform properties. Particularly desirable are uniform density, uniform distribution of the active ingredient, uniform mechanical properties, uniform porosity, uniform appearance of the surface, etc. Only under these circumstances the uniformity of the pharmacological properties, such as the stability of the release profile, may be ensured and the amount of rejects can be kept low.

A further aspect of the invention relates to the use of a pharmacologically active ingredient (A) for the manufacture of the pharmaceutical dosage form as described above for the treatment of pain.

A further aspect of the invention relates to the use of a pharmaceutical dosage form as described above for avoiding or hindering the abuse of the pharmacologically active ingredient (A) contained therein.

A further aspect of the invention relates to the use of a pharmaceutical dosage form as described above for avoiding or hindering the unintentional overdose of the pharmacologically active ingredient (A) contained therein.

In this regard, the invention also relates to the use of a pharmacologically active ingredient (A) as described above and/or a polyalkylene oxide (C) as described above for the manufacture of the pharmaceutical dosage form according to the invention for the prophylaxis and/or the treatment of a disorder, thereby preventing an overdose of the pharmacologically active ingredient (A), particularly due to comminution of the pharmaceutical dosage form by mechanical action.

Further, the invention relates to a method for the prophylaxis and/or the treatment of a disorder comprising the administration of the pharmaceutical dosage form according to the invention, thereby preventing an overdose of the pharmacologically active ingredient (A), particularly due to comminution of the pharmaceutical dosage form by mechanical action. Preferably, the mechanical action is selected from the group consisting of chewing, grinding in a mortar, pounding, and using apparatuses for pulverizing conventional pharmaceutical dosage forms.

The following examples further illustrate the invention but are not to be construed as limiting its scope:

I all examples the dosage forms were tablets assuming a round shape with a diameter of 12 mm.

### General procedure

Polyethylene oxide, α-tocopherol, tramadol hydrochloride and all other excipients were weighted and sieved to each other. The powder was mixed and dosed gravimetrically to an extruder. Hot-melt extrusion was performed by means of a twin screw extruder of type Micro 27 GL 40 D (Leistritz, Nürnberg, Germany) that was equipped with a heatable round die having a diameter of 10 mm.

The hot extrudate was cooled on a conveyor belt and the cooled extrusion strand was comminuted to cut pieces. The cut pieces were shaped by means of an excenter press.

The breaking strength of the pharmaceutical dosage forms was measured by means of a Sotax® HT100 at a constant speed of 120 mm/min and/or a Zwick Z 2.5 at a constant speed of 10 mm/min. A tablet was regarded as failing the breaking strength test when during the measurement the force dropped below the threshold value of 25% of the maximum force that was observed during the measurement, regardless of whether the dosage form was fractured into separate pieces or not. All values are given as mean of 3 measurements (Zwick; n = 3) or as a mean of 10 measurements (Sotax, n = 10).

The in vitro release profile of the pharmacologically active ingredient was measured in 600 ml of artificial gastric juice (pH 6.8) at temperature of 37°C with sinker (type 4). The rotation speed of the paddle was adjusted to 75/min. The pharmacologically active ingredient was detected by means of a spectrometric measurement with a wavelength of 271 nm.

### Example I: Sodium carboxymethylstarch

Tablets having the following composition were prepared:

| Excipient | Reference [mg] | Sample 1 (10%) [mg] | Sample 2 (20%) [mg] |
|---|---|---|---|
| Tramadol - HCl | 80.0 | 80.0 | 80.0 |
| Polyethylenoxide 7,000,000 | 365.8 | 365.8 | 305.8 |
| Polyethylenglycol 6,000 | 90.0 | 90.0 | 90.0 |
| Hypromellose 100,000 mPas | 60.0 | - | - |
| α-Tocopherol | 1.2 | 1.2 | 1.2 |
| Citric Acid | 3.0 | 3.0 | 3.0 |
| Sodium carboxymethylstarch type A | - | 60.0 | 120.0 |

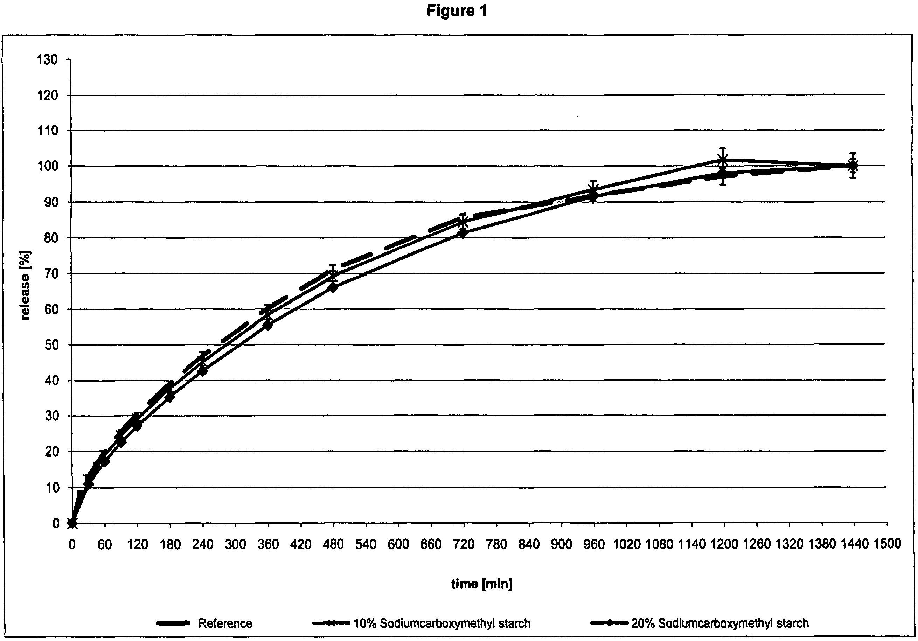

As illustrated in Figure 1, addition of 20% of sodium carboxymethylstarch leads to a retarded release of the pharmaceutical agent.

### Example II: Carmellose Sodium

Tablets having the following composition were prepared:

| Excipient | Reference [mg] | Sample 1 (10%) [mg] | Sample 2 (20%) [mg] | Sample 3 (10%-10%) [mg] |
|---|---|---|---|---|
| Tramadol - HCl | 80.0 | 80.0 | 80.0 | 80.0 |
| Polyethylenoxide 7,000,000 | 365.8 | 365.8 | 305.8 | 305.8 |
| Polyethylenglycol 6,000 | 90.0 | 90.0 | 90.0 | 90.0 |
| Hypromellose 100,000 mPas | 60.0 | - | - | 60.0 |
| α-Tocopherol | 1.2 | 1.2 | 1.2 | 1.2 |
| Citric Acid | 3.0 | 3.0 | 3.0 | 3.0 |
| Carmellose Sodium | - | 60.0 | 120.0 | 60.0 |

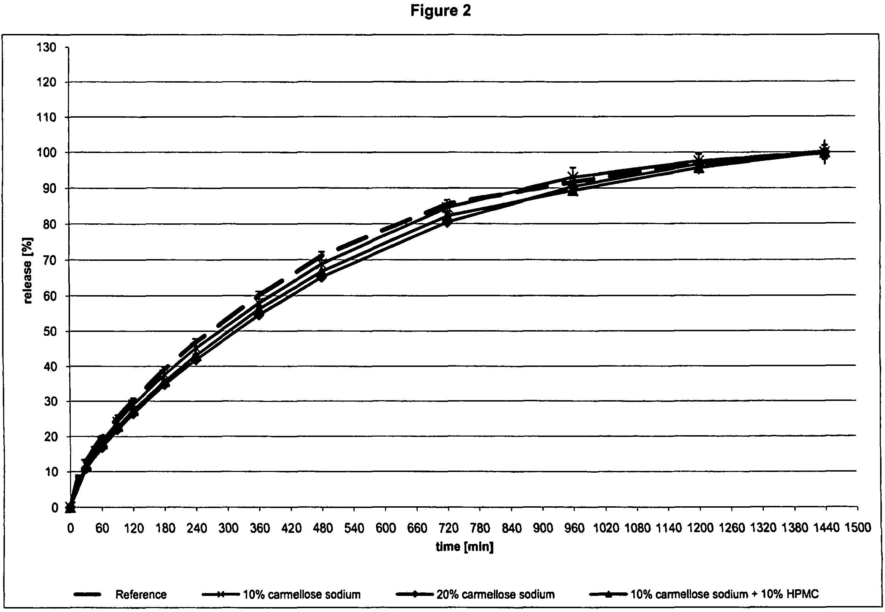

An addition of carmellose sodium leads to a negligible retarded release of the pharmaceutical agent which is illustrated in Figure 2.

### Examples III: Carmellose

Tablets having the following composition were prepared:

| Excipient | Reference [mg] | Sample 1 (10%) [mg] | Sample 2 (20%) [mg] | Sample 3 (10%-10%) [mg] |
|---|---|---|---|---|
| Tramadol - HCl | 80.0 | 80.0 | 80.0 | 80.0 |
| Polyehtylenoxide 7,000,000 | 365.8 | 365.8 | 305.8 | 305.8 |
| Polyethylenglycol 6,000 | 90.0 | 90.0 | 90.0 | 90.0 |
| Hypromellose 100,000 mPas | 60.0 | - | - | 60.0 |
| α-Tocopherol | 1.2 | 1.2 | 1.2 | 1.2 |
| Citric Acid | 3.0 | 3.0 | 3.0 | 3.0 |
| Carmellose | - | 60.0 | 120.0 | 60.0 |

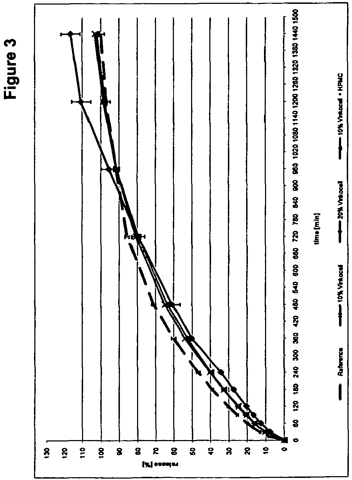

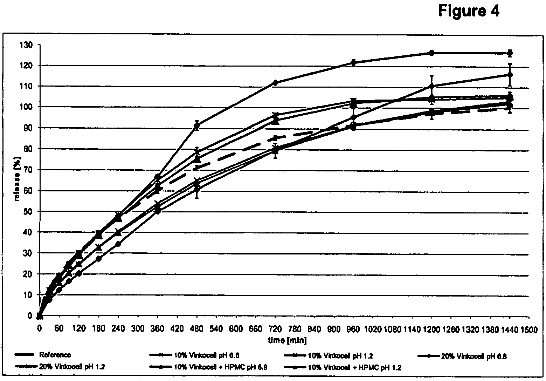

An addition of carmellose leads to a retarded release of the pharmaceutical agent. This fact does not depend on the amount of HPMC in the dosage form which is illustrated in Figure 3. Under acid conditions the mentioned retarded release changes to an acceleration of the release. This is illustrated in Figure 4.

### Example IV: Crosscarmellose Sodium

Tablets having the following composition were prepared:

| Excipient | Reference [mg] | Sample 1 (10%) [mg] | Sample 2 (20%) [mg] | Sample 3 (10%-10%) [mg] |
|---|---|---|---|---|
| Tramadol - HCl | 80.0 | 80.0 | 80.0 | 80.0 |
| Polyehtylenoxide 7,000,000 | 365.8 | 365.8 | 305.8 | 305.8 |
| Polyethylenglycol 6,000 | 90.0 | 90.0 | 90.0 | 90.0 |
| Hypromellose 100,000 mPas | 60.0 | - | - | 60.0 |
| α-Tocopherol | 1.2 | 1.2 | 1.2 | 1.2 |
| Citric Acid | 3.0 | 3.0 | 3.0 | 3.0 |
| Crosscarmellose | - | 60.0 | 120.0 | 60.0 |

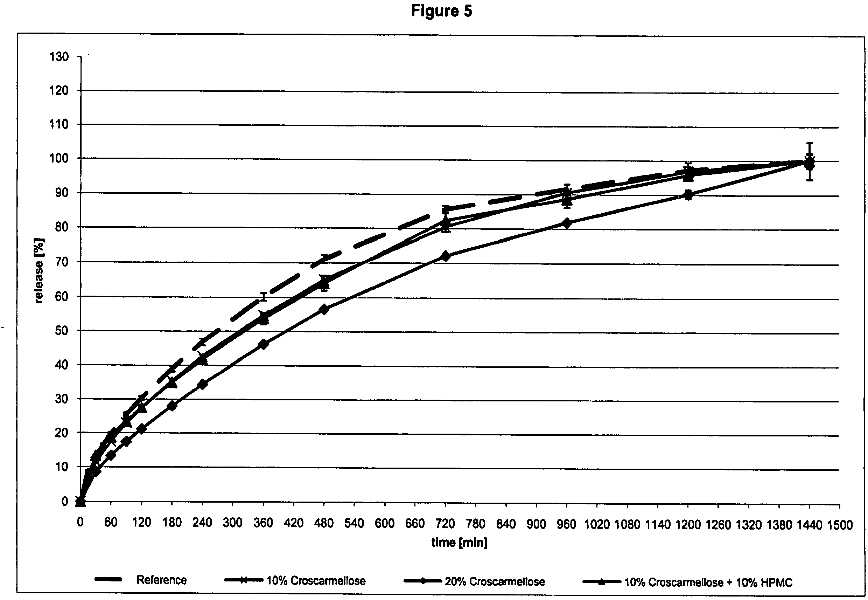

An addition of crosscarmellose sodium leads to a retarded release of the pharmaceutical agent. This fact does not depend on the amount of HPMC in the dosage form and is illustrated in Figure 5.

### Example V: Carrageenan

Tablets having the following composition were prepared:

| Excipient | Reference | | Sample | |
|---|---|---|---|---|
| | mg | % | mg | % |
| Tramadol-HCl | 80.0 | 13.3 | 80.0 | 13.3 |
| Polyethylenoxide 7,000,000 | 370.0 | 61.7 | 370.0 | 61.7 |
| Hypromellose 100,000 | 60.0 | 10.0 | - | - |
| Macrogol 6,000 | 90.0 | 15.0 | 90.0 | 15.0 |
| K-Carrgeenan | - | - | 60.0 | 10.0 |
| Total | 600.0 | - | 600.0 | - |

The batch could be manufactured under standard process conditions. However, tablets showed a slightly deficient surface. The tablets' breaking strengths were tested using a Sotax HT-100 breaking force tester equipped with plain brackets. The mean breaking strength was above the measuring range of the instrument of 1000 N.

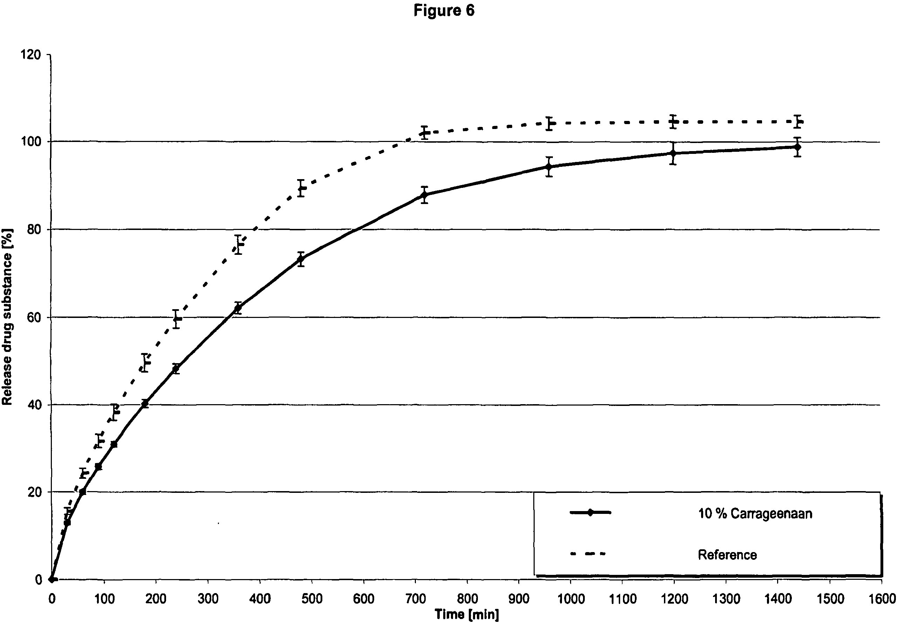

An addition of κ-carrageenan leads to a retarded release of the pharmaceutical agent (37°C, rpm = 75, 600 mL phosphate buffer, pH = 6.8, n = 3 ± s). The dissolution speed of the formulation with Carrageenan is slower than that of the reference composition. A reduction of about 15 % between 500 and 720 minutes could be achieved. This is illustrated in Figure 6.

### Comparative Example I: Cornstarch

Tablets having the following composition were prepared:

| Excipient | Reference [mg] | Sample 1 (10%) [mg] | Sample 2 (20%) [mg] |
|---|---|---|---|
| Tramadol - HCl | 80.0 | 80.0 | 80.0 |
| Polyehtylenoxide 7,000,000 | 365.8 | 365.8 | 305.8 |
| Polyethylenglycol 6,000 | 90.0 | 90.0 | 90.0 |
| Hypromellose 100,000 mPas | 60.0 | - | - |
| α-Tocopherol | 1.2 | 1.2 | 1.2 |
| Citric Acid | 3.0 | 3.0 | 3.0 |
| Corn Starch | - | 60.0 | 120.0 |

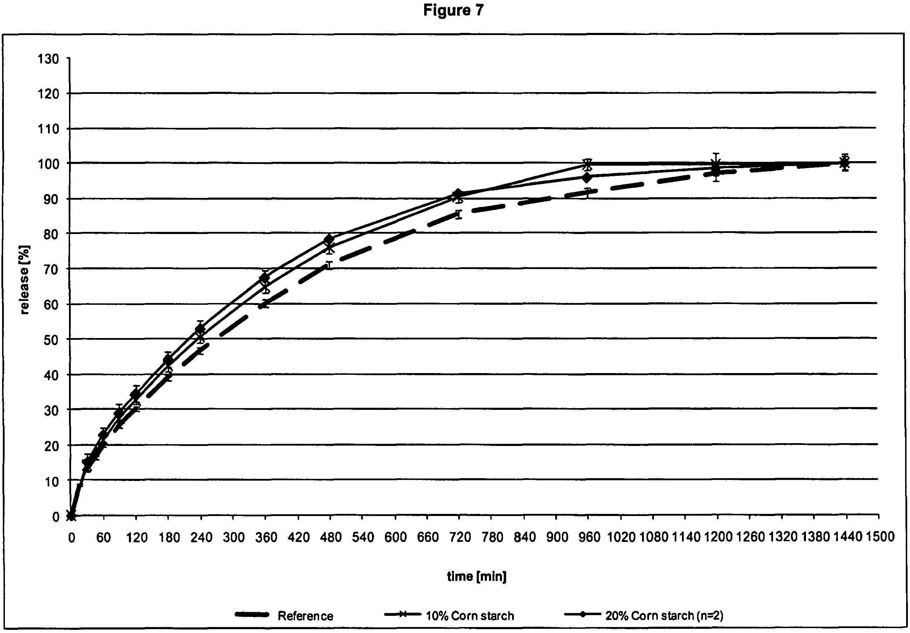

An addition of corn starch increases the release of the pharmaceutical agent. Corn starch acts as a decomposition excipient in this case, which is illustrated in Figure 7.

### Comparative Example II: Pregelatinized Starch.

Tablets having the following composition were prepared:

| Excipient | Reference [mg] | Sample 1 (10%) [mg] | Sample 2 (20%) [mg] |
|---|---|---|---|
| Tramadol - HCl | 80.0 | 80.0 | 80.0 |
| Polyehtylenoxide 7,000,000 | 365.8 | 365.8 | 305.8 |
| Polyethylenglycol 6,000 | 90.0 | 90.0 | 90.0 |
| Hypromellose 100,000 mPas | 60.0 | - | - |
| α-Tocopherol | 1.2 | 1.2 | 1.2 |
| Citric Acid | 3.0 | 3.0 | 3.0 |
| Starch 1500 | - | 60.0 | 120.0 |

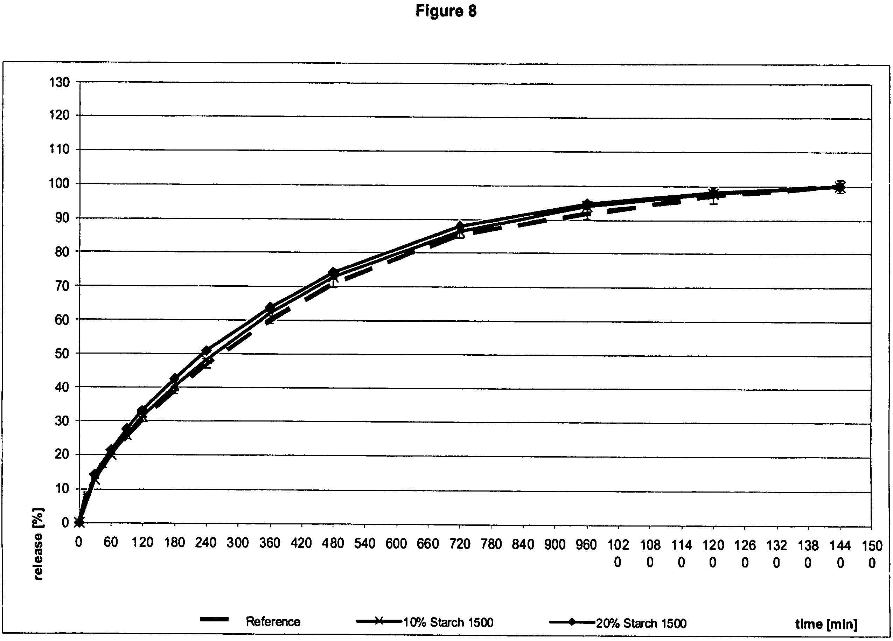

An addition of starch 1500 increases the release of the pharmaceutical agent. Starch 1500 acts as a decomposition excipient in this case, which is illustrated in Figure 8.

### Comparative Example III: - Celluloseacetate

Celluloseacetate is the non-ionic acetate ester of cellulose. Tablets having the following composition were prepared:

| Excipient | Reference | | Sample | |
|---|---|---|---|---|
| | mg | % | mg | % |
| Tramadol-HCl | 80.0 | 13.3 | 80.0 | 13.3 |
| Polyethylenoxide 7,000,000 | 370.0 | 61.7 | 370.0 | 61.7 |
| Hypromellose 100,000 | 60.0 | 10.0 | - | - |
| Macrogol 6,000 | 90.0 | 15.0 | 90.0 | 15.0 |
| Celluloseacetate | - | - | 60.0 | 10.0 |
| Totals | 600.0 | - | 600.0 | - |

During extrusion, the strand showed a slight wavy surface. The breaking strengths were tested using a Sotax HT-100 breaking force tester equipped with plain brackets. The mean breaking strength was 999 N just below the upper limit of the measuring range of the instrument of 1000 N.

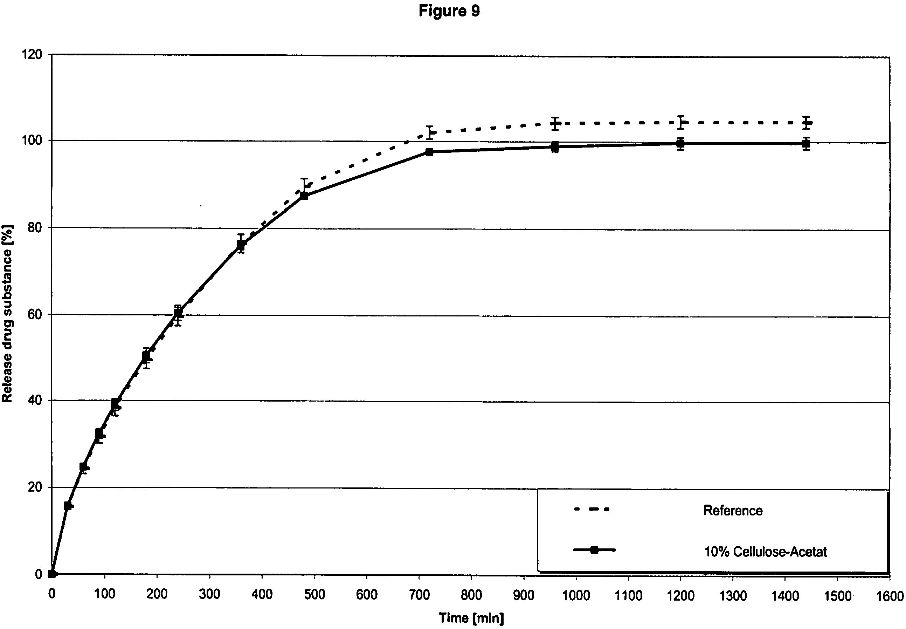

The dissolution speed (37°C, rpm = 75, 600 mL phosphate buffer, pH = 6.8, n = 3 ± s) of the formulation with celluloseacetate is comparable to that of the reference composition, which is illustrated in Figure 9.

### Comparative Example IV: Pectin

Pectin is an herbal heteropolysaccharide containing galacturonic acid side chains. Tablets having the following composition were prepared:

| Excipient | Reference | | Sample | |
|---|---|---|---|---|
| | mg | % | mg | % |
| Tramadol-HCl | 80.0 | 13.3 | 80.0 | 13.3 |
| Polyethylenoxide 7,000,000 | 370.0 | 61.7 | 370.0 | 61.7 |
| Hypromellose 100,000 | 60.0 | 10.0 | - | - |
| Macrogol 6,000 | 90.0 | 15.0 | 90.0 | 15.0 |
| Pectin | - | - | 60.0 | 10.0 |
| Totals | 600.0 | - | 600.0 | - |

No special observations were made during the manufacturing. The breaking strengths of the tablets were tested using a Sotax HT-100 breaking force tester equipped with plain brackets. The mean breaking strength was 999 N just below the upper limit of the measuring range of the instrument of 1000 N.

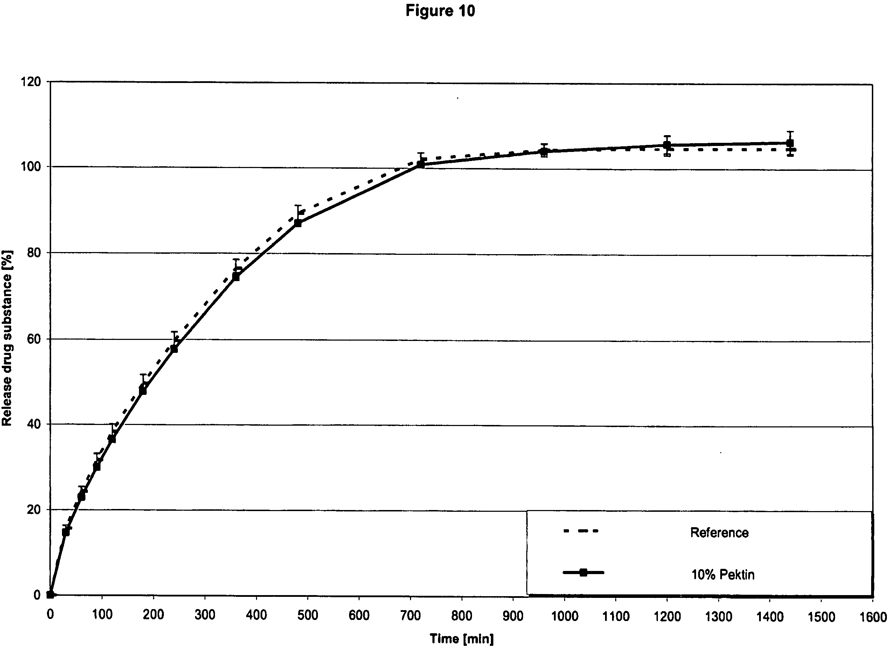

The dissolution speed (37°C, rpm = 75, 600 mL phosphate buffer, pH = 6.8, n = 3 ± s) of the formulation with pectin is identical to that of the reference composition, which is illustrated in Figure 10.

### Comparative Example V: Phosphatized Starch

Di-starch phosphate (E1412) is an acid-treated ("modified") starch that is cross-linked for reasons of rheology modification. Cross-linking is typically achieved by means of POCl₃ or sodium trimetaphosphate. The product used was Clearam Cl 30 00. Tablets having the following composition were prepared:

| Excipient | Reference | | Sample 1 | | Sample 2 | | Sample 3 | |
|---|---|---|---|---|---|---|---|---|
| | mg | % | mg | % | mg | % | mg | % |
| Tramadol-HCl | 80.0 | 13.3 | 80.0 | 13.3 | 80.0 | 13.3 | 80.0 | 13.3 |
| Polyethylenoxide z,000,000 | 365.8 | 61.0 | 365.8 | 61.0 | 305.8 | 51.0 | 305.8 | 51.0 |
| Hypromellose 100,000 | 60.0 | 10.0 | - | - | - | - | 60.0 | 10.0 |
| Clearam CL 30 00 | - | - | 60.0 | 10.0 | 120.0 | 20.0 | 60.0 | 10.0 |
| Macrogol 6,000 | 90.0 | 15.0 | 90.0 | 15.0 | 90.0 | 15.0 | 90.0 | 15.0 |
| α-Tocopherol | 1.2 | 0.2 | 1.2 | 0.2 | 1.2 | 0.2 | 1.2 | 0.2 |
| Citric acid anhydrous | 3.0 | 0.5 | 3.0 | 0.5 | 3.0 | 0.5 | 3.0 | 0.5 |
| Totals | 600.0 | - | 600.0 | - | 600.0 | - | 600.0 | - |

During the manufacturing of the batches a wavy strand surfaces was observed. The breaking strengths of the tablets were tested using a Sotax HT-100 breaking force tester equipped with plain brackets. The mean breaking strength for all batches was above the measuring range of the instrument of 1000 N.

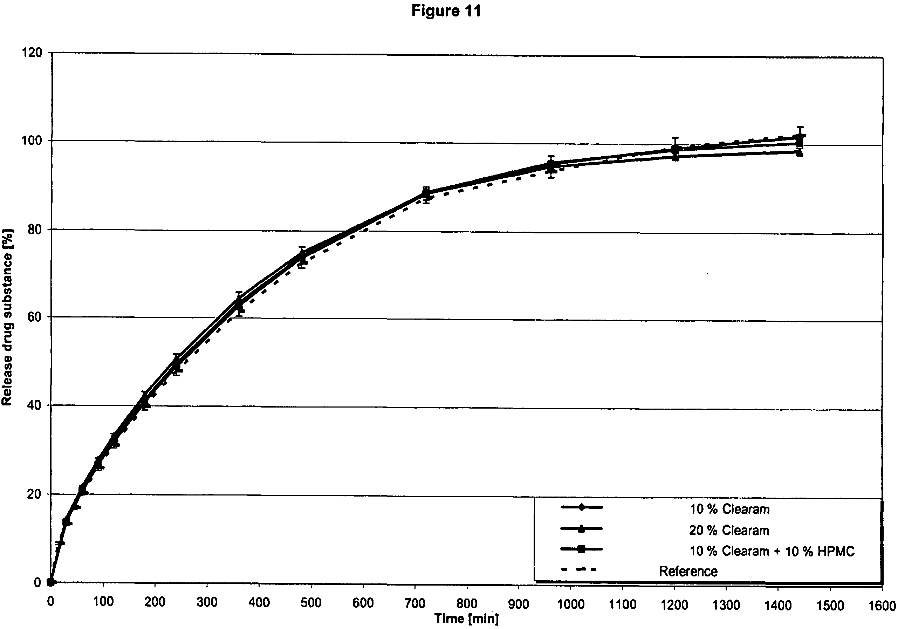

The dissolution speed (37°C, rpm = 75, 600 mL phosphate buffer, pH = 6.8, n = 3 ± s) of all the batches is identical with that of the reference, which is illustrated in Figure 11.

## Claims

1. A pharmaceutical dosage form exhibiting a breaking strength of at least 500 N, said dosage form containing
a pharmacologically active ingredient (A);
an anionic polysaccharide (B) obtainable by introducing anionic functional groups, in protonated form or a physiologically acceptable salt thereof, into a polysaccharide, which is selected from the group consisting of carboxymethylcellulose, carboxymethylstarch, crosscarmellose, and carrageenan; and
a polyalkylene oxide (C);
wherein the pharmacologically active ingredient (A) is present in a controlled-release matrix comprising the anionic polysaccharide (B) and the polyalkylene oxide (C).

2. The pharmaceutical dosage form according to claim 1, which is prepared by hot-melt extrusion.

3. The pharmaceutical dosage form according to any of the preceding claims, which is a tablet.

4. The pharmaceutical dosage form according to any of the preceding claims which has released under *in vitro* conditions:
after 1 h at most 17 wt%
after 2 h at most 32 wt%
after 3 h at most 42 wt%
after 4 h at most 49 wt%
after 7 h at most 68 wt%
after 10 h at most 80 wt%
after 13 h at most 89 wt%
of the pharmacologically active ingredient (A).

5. The pharmaceutical dosage form according to one of the preceding claims, wherein pharmacologically active ingredient (A) is an opioid.

6. The pharmaceutical dosage form according to claim any of the preceding claims, wherein the anionic polysaccharide (B) is selected from the group consisting of carboxymehtyl cellulose sodium, sodium carboxymethylstarch, crosscarmellose, carboxymethyl cellulose and carrageenan.

7. The pharmaceutical dosage form according to any of the proceeding claims, wherein the polyalkylene oxide (C) has a molecular weight of at least 0.5 million g/mol.

8. The pharmaceutical dosage form according to any of the proceeding claims, wherein the polyalkylene oxide (C) has a molecular weight of at least 1 million g/mol.

9. The pharmaceutical dosage form according to any of the proceeding claims, wherein the polyalkylene oxide (C) has a molecular weight within the range of from 1 to 15 million g/mol.

10. The pharmaceutical dosage form according to any of the proceeding claims, wherein the relative weight ratio of the polyalkylene oxide (C) to the anionic polysaccharide (B) is within the range of from 8:1 to 1.5:1.

11. The pharmaceutical dosage form according to any of the proceeding claims, which comprises a further polymer selected from polyglycolides and polyalkylenglycoles.

## Patentansprüche

1. Eine pharmazeutische Darreichungsform, die eine Bruchfestigkeit von wenigstens 500 N aufweist, wobei die Darreichungsform
einen pharmazeutisch aktiven Inhaltsstoff (A),
ein anionisches Polysaccharid (B), das erhältlich ist durch Einführen anionischer funktioneller Gruppen, die in protonierter Form oder als deren physiologisch verträgliche Salze vorliegen, in ein Polysaccharid, das ausgewählt wird aus der Gruppe bestehend aus Carboxymethylcellulose, Carboxymethylstärke, Croscarmellose und Carrageen, und
ein Polyalkylenoxid (C),
enthält;
wobei der pharmazeutisch aktive Inhaltsstoff (A) in der kontrolliert-freisetzenden Matrix vorhanden ist, die das anionische Polysaccharid (B) und das Polyalkylenoxid (C) umfasst.

2. Die pharmazeutische Darreichungsform gemäß Anspruch 1, wobei diese pharmazeutische Darreichungsform durch Hotmelt-Extrusion hergestellt wird.

3. Die pharmazeutische Darreichungsform gemäß einem der vorhergehenden Ansprüche, wobei die pharmazeutische Darreichungsform eine Tablette ist.

4. Die pharmazeutische Darreichungsform gemäß einem der vorhergehenden Ansprüche, die unter *in vitro* Bedingungen
| | |
|---|---|
| nach 1 h | höchstens 17 Gew.-% |
| nach 2 h | höchstens 32 Gew.-% |
| nach 3 h | höchstens 42 Gew.-% |
| nach 4 h | höchstens 49 Gew.-% |
| nach 7 h | höchstens 68 Gew.-% |
| nach 10 h | höchstens 80 Gew.-% |
| nach 13 h | höchstens 89 Gew.-% |
des pharmazeutisch aktiven Inhaltsstoffes (A) freigesetzt hat.

5. Die pharmazeutische Darreichungsform gemäß einem der vorhergehenden Ansprüche, wobei der pharmazeutisch aktive Inhaltsstoff (A) ein Opioid ist.

6. Die pharmazeutische Darreichungsform gemäß einem der vorhergehenden Ansprüche, wobei das anionische Polysaccharid (B) ausgewählt wird aus der Gruppe bestehend aus Natrium-Carboxymethylcellulose, NatriumCarboxymethylstärke, Croscarmellose, Carboxymethylcellulose und Carrageen.

7. Die pharmazeutische Darreichungsform gemäß einem der vorhergehenden Ansprüche, wobei das Polyalkylenoxid (C) ein Molekulargewicht von wenigstens 0,5 Millionen g/mol aufweist.

8. Die pharmazeutische Darreichungsform gemäß einem der vorhergehenden Ansprüche, wobei das Polyalkylenoxid (C) ein Molekulargewicht von wenigstens 1 Millionen g/mol aufweist.

9. Die pharmazeutische Darreichungsform gemäß einem der vorhergehenden Ansprüche, wobei das Polyalkylenoxid (C) ein Molekulargewicht im Bereich von ab 1 bis 15 Millionen g/mol aufweist.

10. Die pharmazeutische Darreichungsform gemäß einem der vorhergehenden Ansprüche, wobei das relative Gewichtsverhältnis des Polyalkylenoxids (C) zum anionischen Polysaccharid (B) innerhalb des Bereichs von ab 8:1 bis 1,5:1 liegt.

11. Die pharmazeutische Darreichungsform gemäß einem der vorhergehenden Ansprüche, die ein weiteres Polymer ausgewählt aus Polyglycoliden und Polyalkylenglycolen umfasst.

## Revendications

1. Forme galénique pharmaceutique présentant une résistance à la rupture d'au moins 500 N, ladite formule galénique contenant
un principe actif pharmacologique (A) ;
un polysaccharide anionique (B) pouvant être obtenu par l'introduction de groupements fonctionnels anioniques, sous forme protonée ou l'un des sels physiologiquement acceptables de celui-ci, dans un polysaccharide, qui est choisi dans le groupe constitué par la carboxyméthylcellulose, le carboxyméthylamidon, le croscarmellose et le carraghénane ; et
un polyoxyde d'alkylène (C) ;
le principe actif pharmacologique (A) étant présent dans une matrice à libération contrôlée comprenant le polysaccharide anionique (B) et le polyoxyde d'alkylène (C).

2. Forme galénique pharmaceutique selon la revendication 1, qui est élaborée par extrusion à l'état fondu.

3. Forme galénique pharmaceutique selon l'une quelconque des revendications précédentes, qui est un comprimé.

4. Forme galénique pharmaceutique selon l'une quelconque des revendications précédentes, qui libère dans les conditions *in vitro* suivantes :
après 1 heure au maximum 17 % en masse
après 2 heures au maximum 32 % en masse
après 3 heures au maximum 42 % en masse
après 4 heures au maximum 49 % en masse
après 7 heures au maximum 68 % en masse
après 10 heures au maximum 80 % en masse
après 13 heures au maximum 89 % en masse du principe actif pharmacologique (A).

5. Forme galénique pharmaceutique selon l'une des revendications précédentes, où le principe actif pharmacologique (A) est un opioïde.

6. Forme galénique pharmaceutique selon l'une quelconque des revendications précédentes, où le polysaccharide anionique (B) est choisi dans le groupe constitué par la carboxyméthylcellulose sodium, le carboxyméthylamidon sodium, le croscarmellose, la carboxyméthylcellulose et le carraghénane.

7. Forme galénique pharmaceutique selon l'une quelconque des revendications précédentes, où le polyoxyde d'alkylène (C) présente une masse moléculaire d'au moins 0,5 million de g/mol.

8. Forme galénique pharmaceutique selon l'une quelconque des revendications précédentes, où le polyoxyde d'alkylène (C) présente une masse moléculaire d'au moins 1 million de g/mol.

9. Forme galénique pharmaceutique selon l'une quelconque des revendications précédentes, où le polyoxyde d'alkylène (C) présente une masse moléculaire située dans un intervalle compris entre entre 1 et 15 millions de g/mol.

10. Forme galénique pharmaceutique selon l'une quelconque des revendications précédentes, où le rapport massique relatif du polyoxyde d'alkylène (C) sur le polysaccharide anionique (B) est situé dans un intervalle compris entre 8:1 et 1,5:1.

11. Forme galénique pharmaceutique selon l'une quelconque des revendications précédentes, qui comprend un polymère supplémentaire choisi parmi les polyglycolides et les polyalkylène glycols.
